# EUROPÄISCHE PATENTSCHRIFT

(11) **EP 3 523 107 B1**
(45) Veröffentlichungstag und Bekanntmachung des Hinweises auf die Patenterteilung: **16.06.2021**
(21) Anmeldenummer: 17780690.8
(22) Anmeldetag: 29.09.2017
(51) Int. Cl.: B29C 45/16, A61M 5/31

(54) **VERFAHREN ZUM HERSTELLEN EINER SPRITZE MIT EINEM INTEGRIERTEN VERSCHLUSSELEMENT**
METHOD OF MANUFACTURING A SYRINGE WITH AN INTEGRATED CLOSURE ELEMENT
PROCÉDÉ DE FABRICATION D'UNE SERINGUE UN ÉLÉMENT DE FERMETURE INTÉGRÉS

(30) Priorität: 04.10.2016 DE 102016118768; 04.10.2016 DE 102016118767
(43) Veröffentlichungstag der Anmeldung: 14.08.2019
(73) Patentinhaber: Gerresheimer Regensburg GmbH, 93047 Regensburg (DE)
(72) Erfinder: VOGL, Maximilian, 92708 Mantel (DE); KIRSCHNER, Ulf, 92533 Wernberg-Köblitz (DE); KREHER, Jessica, 92421 Schwandorf (DE)
(74) Vertreter: Hannke, Christian
(86) Internationale Anmeldenummer: PCT/EP2017/074788
(87) Internationale Veröffentlichungsnummer: WO 2018/065312

(56) Entgegenhaltungen:
- EP-A1- 0 073 356
- EP-A1- 0 849 173
- EP-A1- 1 024 091
- EP-A1- 1 600 190
- DE-T2- 69 333 607
- JP-A- 2003 311 780
- JP-A- 2004 329 504
- US-A1- 2008 102 235
- Anonym: "Entwicklungsprojekt TELC in Zusammenarbeit zwischen den Firmen Gerresheimer Bünde GmbH und der Braunform GmbH", reinaum online, 10. März 2013 (2013-03-10), Seite 1, XP055439689, Gefunden im Internet: URL:http://www.braunform.com/de/downloads/ Veroeffentlichungen/ältere/2013_Reinraum-p rintline_TELC.pdf [gefunden am 2018-01-10]

## Beschreibung

Die vorliegende Erfindung betrifft ein Spritzgießverfahren zum Herstellen einer Spritze mit einem integrierten Verschlusselement, sowie eine Spritze mit einem integrierten Verschlusselement.

Die Anwendung sogenannter vorgefüllter Kunststoffspritzen ist in der Medizin zwischenzeitlich weit verbreitet. Diese Spritzen werden bereits mit dem zur Anwendung kommenden Medium an den Nutzer ausgeliefert und haben den Vorteil, dass deren Handhabung sehr einfach ist, da das Medium nicht vor der Anwendung in die Spritze transferiert werden muss. Weiterhin ist, selbst im Notfall, die Wahrscheinlichkeit der Anwendung eines falschen Medikaments sehr gering. Derartige Spritzen finden ebenso Anwendung in der Pharma- und Biotech-Industrie. Für Impfstoffe und zahlreiche andere Medikamente sind sie heutzutage das Primärpackmittel erster Wahl.

In der Regel weisen solche Spritzen ein Endstück auf, welches als ein Konus, dessen Außendurchmesser sich kontinuierlich verjüngt, ausgebildet ist. Solche Koni sind überwiegend als ein männlicher Luer-Konus ausgebildet. Ein Luer-Ansatz, -Steck oder -Slip System ist ein genormtes (ISO-Norm 594) Verbindungssystem für Schlauchsysteme im medizinischen Bereich und garantiert die Kompatibilität zwischen verschiedenen Herstellern. Es findet unter anderem Anwendung bei Spritzen, Kanülen, und Infusionsschläuchen. Ein männlicher Luer-Konus ist ein flacher (Steigung 6%) Hohlkegel mit relativ großer Oberfläche und daher guter Haftung mit einem aufgesteckten (weiblichen) Luer-Ansatz von beispielsweise einer Kanüle. Durch die kegelförmige Konstruktion beider Verbindungsteile wird eine ausreichende Dichtung erreicht.

Oft ist es notwendig die Verbindung einer Spritze mit einem Transfersystem, beispielsweise einem Schlauch oder Ähnlichem, gegen ein unerwünschtes Lösen zu sichern. Dazu werden die Spritzen mit einem Anschlusselement ausgestattet, welches ein Innengewinde aufweist und somit oft auch als Überwurfmutter bezeichnet wird. Mittels des Innengewindes kann die Spritze reversibel mit entsprechenden Transfersystemen verbunden werden, wodurch ein unerwünschtes Lösen der Verbindung verhindert wird. Um die Kompatibilität mit einer größtmöglichen Anzahl an Anschlusselementen zu gewährleisten werden das Anschlusselement beziehungsweise das Innengewinde genormt ausgebildet. Ein solches genormtes Verschraubungssystem ist das Luer-Lock-System. Die Luer-Lock- Verbindung hat sich weltweit für reversible Verbindungen von Spritzen, Kanülen, Infusionsschläuchen, Spinalnadeln, etc. durchgesetzt.

Die vorgefüllten Spritzen können im Nachhinein mit aufsetzbaren Luer-Lock-Anschlüssen ausgestattet werden. Es gibt aber auch Spritzenkörper, welche bereits mit einem Luer-Lock-Anschluss hergestellt werden. Solche vorfüllbare Spritzen(-körper) werden von dem Primärpackmittelhersteller unter Reinraumbedingungen hergestellt, abgepackt, sterilisiert und abfüllfertig direkt in den Reinraum des Abfüllers, beispielsweise ein Pharmazeut, geliefert. Sie können dann ohne weitere Behandlungsschritte zur Abfüllung eingesetzt werden. Meist werden die Innenwände der Spritzen auch silikonisiert, damit der Kolbenstopfen sich in der Spritze leicht bewegt und trotzdem eine ausreichende Dichtigkeit zwischen Spritzenkörper und Kolben gewährleistet ist. Die vorfüllbaren Spritzen werden durch den Abfüller von der proximalen Seite, bzw. der Flanschseite befüllt. Demzufolge muss das distale Ende der Spritze vorher verschlossen sein. In der Regel ist dies ein elastischer, dichtender Verschluss, welcher vor der Verwendung der Spritze entfernt wird. Dieser Verschluss kann sowohl aus einem Gummi hergestellt werden oder aus einem elastischen Thermoplasten. Im bisherigen bekannten Stand der Technik wird ein solcher Verschluss bei der Produktion an der Spritze montiert. Ein Nachteil einer solchen nachträglich zu integrierenden Montage eines Verschlusses sind höhere Aufwendungen für die Automatisierung. Dies verursacht zum einen Kosten durch die zusätzlich notwendigen aufwändigen Gerätschaften wie beispielsweise Roboterarme. Ebenso werden die Zykluszeiten für die Herstellung einer einzelnen Spritze verlängert. Zusätzlich besteht die erhöhte Gefahr einer Verunreinigung des Kopfbereichs der Spritze, da diese zu Beginn der Verarbeitung offen ist und erst nachträglich verschlossen wird. Verfahren zur Herstellung von Spritzen, Karpulen oder anderen Artikeln mit Anschluss- und Verschlusselementen sind aus DE 693 33 607 T2, EP 1 600 190 A1, JP 2004 329504 A, JP 2003 311780 A, EP 0 849 173 A1, US 2008/102235 A1, EP 1 024 091 A1 und EP 0 073 356 A1 bekannt.

Aufgabe der vorliegenden Erfindung ist es ein Verfahren zur Herstellung einer vorfüllbaren Kunststoffspritze zur Verfügung zu stellen, welches die eingangs genannten Probleme löst und demnach eine einfache und kostengünstige Herstellung einer solchen Spritze ermöglicht.

Die Aufgabe wird durch ein Verfahren gemäß Patentanspruch 1 gelöst. Demnach wird ein-Spritzgießverfahren zum Herstellen einer Spritze mit einem integrierten Verschlusselement zur Verfügung gestellt, welches die folgenden Verfahrensschritte umfasst:
a) Bereitstellen eines Spritzgusswerkzeugs, welches einen ersten, einen zweiten und einen dritten Werkzeugabschnitt umfasst, wobei der erste Werkzeugabschnitt einen sich entlang einer axialen Richtung (X) erstreckenden beidseitig offenen Formhohlraum aufweist und wobei der zweite Werkzeugabschnitt einen ersten Spritzgusskern und der dritte Werkzeugabschnitt einen zweiten Spritzgusskern aufweist;
b) Schließen des Spritzgusswerkzeugs, so dass der erste Werkzeugabschnitt den zweiten und dritten Werkzeugabschnitt kontaktiert und der erste und zweite Spritzgusskern durch jeweils eine Öffnung in den Formhohlraum des ersten Werkzeugabschnitts in diesen eintreten und sich schließlich kontaktieren, wodurch diese Werkzeugabschnitte eine erste Kavität bilden;
c) Einspritzen eines ersten Kunststoffmaterials in die erste Kavität, wodurch ein hohlzylindrischer Spritzenkörper, an dessen distalen Ende ein Endbereich angeordnet ist, ausgebildet wird, wobei der Endbereich ein mit einem Innengewinde versehenes Anschlusselement und ein hohlzylindrisches Endstück, welches von dem Anschlusselement zumindest abschnittsweise umrandet ist, aufweist;
d) Abkühlen der Werkzeugabschnitte, wodurch der Spritzenkörper abkühlt und aushärtet;
e) Kontaktieren des ersten Werkzeugabschnitts mit einen einseitig geschlossenen Formhohlraum versehenen vierten Werkzeugabschnitt, wodurch eine zweite Kavität an dem distalen Ende des Spritzenkörpers gebildet wird;
f) Einspritzen eines zweiten Kunststoffmaterials in die zweite Kavität, wodurch das Verschlusselement an das Anschlusselement angeformt wird, wobei das erste und das zweite Kunststoffmaterial keine stoffschlüssige Verbindung eingehen.

Durch das erfindungsgemäße Herstellungsverfahren wird eine Spritze mit einem Anschlusselement, welches vorzugsweise als ein Luer-Lock-System ausgestaltet ist zur Verfügung gestellt. Die Spritze ist mit einem integrierten Verschlusselement versehen, welches durch das erfindungsgemäße Verfahren direkt an das Anschlusselement abgeformt wird. Dadurch, dass die beiden Kunststoffmaterialien von Anschlusselement und Verschlusselement keine stoffschlüssige Verbindung eingehen, ist das Verschlusselement von dem Anschlusselement lösbar. In Schritt b) wird ein mit einem Innengewinde versehenes Anschlusselement gebildet, an welches in Schritt f) das Verschlusselement angeformt wird. Folglich wird das Verschlusselement mit einem Außengewinde versehen, da das Innengewinde als formgebende Struktur wirkt. Dieses Außengewinde greift demnach in das Innengewinde des Anschlusselements ein. Dadurch, dass die beiden Kunststoffmaterialien von Anschlusselement und Verschlusselement keine stoffschlüssige Verbindung eingehen sind das Anschlusselement und Verschlusselement form- beziehungsweise kraftschlüssig mittels einer Schraubverbindung verbunden. Der Nutzer kann das Verschlusselement somit durch eine Drehbewegung von dem Anschlusselement lösen. Nach einer bevorzugten Ausführungsform ist der vierte Werkzeugabschnitt in Schritt e) erhitzt.

Das Abkühlen der Werkzeugabschnitte beziehungsweise das Abkühlen und Aushärten des Spritzenkörpers ist notwendig um zu gewährleisten, dass das erste und zweite Kunststoffmaterial keine stoffschlüssige Verbindung eingehen. Der Spritzenkörper sollte dabei auf eine Temperatur abgekühlt werden, welche klein genug ist, dass das Einspritzen des zweiten Kunststoffmaterials kein Aufschmelzen der kontaktierten Oberflächen des Spritzenkörpers verursacht.

Das erfindungsgemäße Verfahren ist im Vergleich zu den aus dem Stand der Technik bekannten Verfahren wesentlich einfacher und effektiver, da eine nachträgliche Montage eines Verschlusses entfällt. Weiterhin ist es nicht mehr notwendig das Verschlussteil in einem separaten Herstellungsschritt zu produzieren, wodurch sich der Produktionsaufwand und der Materialaufwand reduzieren. Die erfindungsgemäße Spritze ist somit ökonomischer und einfacher zu produzieren, da zusätzliche Kosten für Herstellung und Montage eines Verschlussteils entfallen. Ferner entfällt die Gefahr einer Verunreinigung des Kopfbereichs der Spritze bevor die Spritze verschlossen wird, da es möglich ist das Verschlusselement in dem gleichen Produktionsschritt wie den Spritzenkörper herzustellen.

Gemäß zumindest einer Ausführungsform weist der Endbereich ein dem einem Innengewinde und wenigstens einer Aussparung versehenes Anschlusselement auf.

Gemäß zumindest einer Ausführungsform wird durch das Einspritzen des zweiten Kunststoffmaterials in die zweite Kavität das Verschlusselement an das Anschlusselement angeformt, wodurch in der wenigstens einen Aussparung ein Vorsprung ausgebildet wird.

Vorzugsweise erstrecken der erste und zweite Spritzgusskern sich entlang der axialen Richtung (X). Bevorzugt weist der erste Spritzgusskern einen ersten zylindrischen Abschnitt mit einem ersten Durchmesser und einen zweiten zylindrischen Abschnitt mit einem zweiten kleineren Durchmesser auf. Vorzugsweise sind der erste und der zweite zylindrische Abschnitt in axialer Richtung (X) angrenzend. Nach einer bevorzugten Ausführungsform weist der zweite Spritzgusskern einen in axialer Richtung (X) einseitig offenen Formholraum auf. Vorteilhafterweise weist der zweite Spritzgusskern weiterhin eine Ausnehmung an einer den Formholraum in axialer Richtung (X) begrenzenden Innenwand auf.

Nach einer bevorzugten Ausführungsform bilden in Verfahrensschritt b) eine innere Wandung des Formhohlraums des ersten Werkzeugabschnitts und eine äußere Wandung des ersten zylindrischen Abschnitts des ersten Spritzgusskerns einen ersten Abschnitt der ersten Kavität, mittels welchem der hohlzylindrischen Spritzenkörper gebildet wird. Bevorzugt wird der Spritzgusskern dabei zentrisch in den Formhohlraum eingebracht, so dass ein Abstand zwischen der inneren Wandung des Formhohlraums des ersten Werkzeugabschnitts und der äußeren Wandung des ersten zylindrischen Abschnitts in jeder radialer Richtung konstant gleich ist. Somit wird eine konstante Wandstärke des Spritzenzylinders gewährleistet.

Vorzugsweise wird bei dem Kontaktieren der beiden Spritzgusskerne in Verfahrensschritt b) der zweite zylindrische Abschnitt des ersten Spritzgusskerns in der Ausnehmung des zweiten Spritzgusskerns teilweise aufgenommen. Vorteilhafterweise wird durch den Formholraum des zweiten Spritzgusskerns und den zweiten zylindrischen Abschnitt des ersten Spritzgusskerns eine dritte Kavität gebildet, mittels welcher das vorzugsweis konisch ausgebildete Endstück ausgebildet wird. Bevorzugt begrenzen dabei eine äußere Wandung des zweiten zylindrischen Abschnitts und eine innere Wandung des Formholraums des zweiten Spritzgusskerns diese dritte Kavität.

Der zweite zylindrische Abschnitt bildet somit den Kanal des Endstücks aus. Dabei ist es vorteilhaft, dass der zweite zylindrische Abschnitt in der Ausnehmung, der den Formhohlraum axial begrenzenden Innenwand, aufgenommen ist. Dadurch ragt der zweite zylindrische Abschnitt über diese Innenwand, welche das distale Ende des Endstücks formt, hinaus. Durch diese Ausgestaltung wird gewährleistet, dass in den Kanal des Endstücks kein erstes Kunststoffmaterial eintritt und diesen somit verschließt. Auch hier ist es vorteilhaft, wenn der zweite zylindrische Abschnitt zentrisch in den Formholraum des zweiten Spritzgusskerns eingeführt wird, um eine konstante Wandstärke des Endstücks zu gewährleisten. Vorzugsweise ist der Formholraum des zweiten Spritzgusskerns konisch ausgestaltet, so dass ein konisches Endstück gebildet werden kann.

Gemäß zumindest einer Ausführungsform umfasst das hier vorgeschlagene Verfahren auch eine zweite Alternative:
a) Bereitstellen eines Spritzgusswerkzeugs, welches einen ersten, einen zweiten und einen dritten Werkzeugabschnitt umfasst, wobei der erste Werkzeugabschnitt einen sich entlang einer ersten axialen Richtung erstreckenden beidseitig offenen Formhohlraum aufweist und wobei der zweite Werkzeugabschnitt einen ersten Spritzgusskern und der dritte Werkzeugabschnitt einen zweiten Spritzgusskern aufweist;
b) Schließen des Spritzgusswerkzeugs, so dass der erste Werkzeugabschnitt den zweiten und dritten Werkzeugabschnitt kontaktiert und der erste und zweite Spritzgusskern durch jeweils eine Öffnung in den Formhohlraum des ersten Werkzeugabschnitts in diesen eintreten und sich schließlich kontaktieren, wodurch diese Werkzeugabschnitte eine erste Kavität bilden;
c) Einspritzen eines ersten Kunststoffmaterials in die erste Kavität, wodurch ein hohlzylindrischer Spritzenkörper, an dessen distalen Ende ein Endbereich angeordnet ist, ausgebildet wird, wobei der Endbereich ein mit einem Innengewinde und wenigstens einer Aussparung versehenes Anschlusselement und ein hohlzylindrisches Endstück, welches von dem Anschlusselement zumindest abschnittsweise umrandet ist, aufweist;
d) Abkühlen der Werkzeugabschnitte, wodurch der Spritzenkörper abkühlt und aushärtet;
e) Kontaktieren des ersten Werkzeugabschnitts mit einem, einen einseitig geschlossenen Formhohlraum aufweisenden vierten Werkzeugabschnitt, wodurch eine zweite Kavität an dem distalen Ende des Spritzenkörpers gebildet wird;
f) Einspritzen eines zweiten Kunststoffmaterials in die zweite Kavität, wodurch das Verschlusselement an das Anschlusselement angeformt wird, wodurch in der wenigstens einen Aussparung ein Vorsprung ausgebildet wird, wobei das erste und das zweite Kunststoffmaterial keine stoffschlüssige Verbindung eingehen.

Durch das in der obigen Alternative vorschlagene Herstellungsverfahren wird eine Spritze mit einem Anschlusselement, welches vorzugsweise als ein Luer-Lock-System ausgestaltet ist zur Verfügung gestellt. Die Spritze ist mit einem integrierten Verschlusselement versehen, welches durch das erfindungsgemäße Verfahren direkt an das Anschlusselement abgeformt wird. Dadurch, dass die beiden Kunststoffmaterialien von Anschlusselement und Verschlusselement keine stoffschlüssige Verbindung eingehen, ist das Verschlusselement von dem Anschlusselement lösbar. In Schritt b) wird ein mit einem Innengewinde sowie mit wenigstens einer Aussparung versehenes Anschlusselement gebildet, an welches in Schritt f) das Verschlusselement angeformt wird. Folglich wird das Verschlusselement mit einem Außengewinde sowie mit wenigstens einem Vorsprung versehen, da das Innengewinde und die Aussparung als formgebende Strukturen wirken. Dieses Außengewinde greift demnach in das Innengewinde des Anschlusselements und der wenigstens eine Vorsprung in die Aussparung des Anschlusselements ein. Dieser Vorsprung stellt ein so genanntes "Tamper Evidence Feature" dar. Unter einem "Tamper Evidence Feature" werden in diesem Zusammenhang alle Elemente subsummiert, welche dazu in der Lage sind anzuzeigen, ob ein Gegenstand, insbesondere eine Spritze, bereits verwendet wurden, so dass aufgrund der Beschaffenheit dieser Elemente der Originalitätszustand eines Produkts offensichtlich erkennbar ist. Das erste und das zweite Kunststoffmaterial liegen dabei in einem flüssigen Zustand vor.

Dadurch, dass die beiden Kunststoffmaterialien von Anschlusselement und Verschlusselement keine stoffschlüssige Verbindung eingehen, sind das Anschlusselement und Verschlusselement form- beziehungsweise kraftschlüssig mittels einer Schraubverbindung verbunden. Der Nutzer kann das Verschlusselement somit durch eine Drehbewegung des Verschlusselements von dem Anschlusselement lösen. Durch das Drehen des Verschlusselements werden die daran angerodneten Vorsprünge beim Austreten aus den Aussparungen irreversibel verformt, so dass das Verschlusselement anzeigt, ob die Spritze bereits verwendet wurde.

Durch das Abkühlen und Aushärten des Spritzenkörpers in Verfahrensschritt d) wird sichergestellt, dass das erste und das zweite Kunststoffmaterial keine stoffschlüssige Verbindung eingehen. Der Spritzenkörper sollte dabei auf eine Temperatur abgekühlt werden, welche klein genug ist, dass das Einspritzen des zweiten Kunststoffmaterials kein Aufschmelzen der kontaktierten Oberflächen des Spritzenkörpers verursacht.

Das erfindungsgemäße Verfahren ist im Vergleich zu den aus dem Stand der Technik bekannten Verfahren wesentlich einfacher und effektiver, da eine nachträgliche Montage eines Verschlusses entfällt. Weiterhin ist es nicht mehr notwendig, das Verschlussteil in einem separaten Herstellungsschritt zu produzieren, wodurch sich der Produktionsaufwand und der Materialaufwand reduzieren. Die erfindungsgemäße Spritze ist somit ökonomischer und einfacher zu produzieren, da zusätzliche Kosten für Herstellung und Montage eines Verschlussteils entfallen. Ferner entfällt die Gefahr einer Verunreinigung des Kopfbereichs der Spritze bevor die Spritze verschlossen wird, da es möglich ist, das Verschlusselement in demselben Verfahren wie den Spritzenkörper herzustellen. Zudem wird durch die Ausbildung des wenigstens einen Vorsprungs ein "Tamper Evidence Feature" direkt an dem Produkt generiert, was eine erhöhte Sicherheit im Hinblick auf Missbrauch oder irrtümlicher erneuter Benutzung liefert, da direkt am Produkt selbst erkennbar ist, ob das Produkt bereits verwendet wurde. Zudem entfällt durch die ausgebildeten Vorsprünge die Notwendigkeit einer Verpackung mit zusätzlichen "Tamper Evidence Features".

Bevorzugt ist die zumindest eine Aussparung von einem äußeren und einem inneren Wandungsabschnitt umrandet, wobei der äußere Wandungsabschnitt einen Teil der Außenfläche des Anschlusselements bildet. Somit befindet sich die Aussparung in radialer Richtung zwischen zwei Wandungsabschnitten. Die wenigstens eine Aussparung ist in der Umfangsrichtung derart ausgestaltet, so dass das Herausdrehen des Verschlusselements ermöglicht und gleichzeitig ein Verformen des wenignstens einen Vorsprungs gewährleistet wird.

Vorzugsweise entspricht die Steigung des Innengewindes des Anschlusselements der Neigung des wenigstens einen Vorsprungs. Dies ist notwendig, um ein Herausdrehen des Verschlusselements zu ermöglichen, ohne dass das Verschlusselement durch den wenigstens einen Vorsprung in dem Anschlusselement verkantet.

Gemäß einer weiteren Ausführungsform wird das mit dem Innengewinde versehene Anschlusselement mittels einer vierten Kavität gebildet, welche durch einen Außenbereich des zweiten Spritzgusskerns und der inneren Wandung des Formhohlraums des ersten Werkzeugabschnitts gebildet wird. Vorzugsweise weist der Außenbereich des zweiten Spritzgusskerns Ausnehmungen und/oder Vorsprünge auf, durch welche das Innengewinde an dem Anschlusselement ausgebildet wird.

Vorzugsweise wird die in Verfahrensschritt e) gebildete zweite Kavität durch eine innere Wandung des Formhohlraums des vierten Werkzeugabschnitts und durch Außenbereiche des Endbereichs des Spritzenkörpers gebildet. Diese Außenbereiche sind vorzugsweise die äußere Wandung des Endstücks und die innere Wandung beziehungsweise das Innengewinde des Anschlusselements. Nach einer bevorzugten Ausführungsform sind an der inneren Wandung des Formhohlraums des vierten Werkzeugabschnitts Ausnehmungen und/oder Vorsprünge angeordnet, so dass das Verschlusselement mit entsprechenden Vorsprüngen, beispielsweise Längsrippen, gebildet wird. Solche Vorsprünge dienen dazu die Haptik des Verschlusselements zu verbessern beziehungsweise bilden diese Vorsprünge einen Griffansatz zum Abdrehen des Verschlusselements.

Vorteilhafterweise wird zwischen den Verfahrensschritten d) und e) der zweite Spritzgusskern von dem Anschlusselement des Spritzenkörpers entformt. Bevorzugt wird der zweite Spritzgusskern durch eine Drehbewegung von dem Innengewinde des Anschlusselements entformt. Vorteilhafterweise wird die Drehbewegung des zweiten Spritzgusskerns durch einen Elektromotor, einen hydraulischen Antrieb oder einen anderweitigen Antrieb angetrieben.

Vorzugsweise wird zwischen den Verfahrensschritten d) und e) der dritte Werkzeugabschnitt von dem ersten Werkzeugabschnitt entfernt. Dies kann beispielsweise durch eine Relativbewegung vom ersten und dritten Werkzeugabschnitt zueinander erfolgen. Eine solche Relativbewegung könnte eine Drehbewegung oder eine translatorische Bewegung sein. Bevorzugt werden auch in Verfahrensschritt e) der erste und der vierte Werkzeugabschnitt relativ zueinander bewegt, so dass diese kontaktieren beziehungsweise aneinander anliegen. Diese Drehbewegung kann auch eine Drehbewegung oder eine translatorische Bewegung sein.

Nach einer weiteren bevorzugten Ausführungsform wird das erste Kunststoffmaterial durch eine erste Einspritzdüse, welche in dem zweiten Werkzeugabschnitt angeordnet ist, eingespritzt. Es wäre allerdings auch denkbar die erste Einspritzdüse in dem ersten Werkzeugabschnitt anzuordnen. Ferner ist es vorteilhaft, dass das zweite Kunststoffmaterial durch eine zweite Einspritzdüse, welche in dem vierten Werkzeugabschnitt angeordnet ist eingespritzt wird.

Vorzugsweise weist der Formhohlraum des ersten Werkzeugabschnitts eine erste Öffnung auf, in welche der erste Spritzgusskern in Schritt b) eintritt. Vorteilhaft ist diese erste Öffnung in dem Bereich der ersten Kavität angeordnet. welche das proximale Ende des Spritzenkörpers bildet. Bevorzugt weist der Formhohlraum des ersten Werkzeugabschnitts eine zweite Öffnung auf, in welche der zweite Spritzgusskern in Verfahrensschritt b) eintritt.

Nach einer bevorzugten Ausführungsform weist der zweite Werkzeugabschnitt eine Ausnehmung auf, welche zur ersten Kavität hin offen ist und mittels welcher eine Fingerauflage am proximalen Ende des Spritzenkörpers gebildet wird. Vorzugsweise ist die erste Einspritzdüse mit dieser Ausnehmung verbunden.

Vorzugsweise ist der den ersten Spritzgusskern aufweisende zweite Werkzeugabschnitt zumindest abschnittsweise T-förmig ausgestaltet. Vorzugsweise weisen sowohl der erste als auch der zweite Werkzeugabschnitt Stirnflächen auf, die sich entlang einer zweiten Richtung (Y), welche senkrecht zu der axialen Richtung verläuft, erstrecken. Vorzugsweise liegen nach Verfahrensschritt b) diese Stirnflächen fest aneinander an. Vorzugsweise wird die erste Kavität, beziehungsweise die erste Öffnung des Formhohlraums des ersten Werkzeugabschnitts in axialer Richtung durch den zweiten Werkzeugabschnitt abgeschlossen.

Vorzugsweise ist der den zweiten Spritzgusskern aufweisende dritte Werkzeugabschnitt zumindest abschnittsweise T-förmig ausgestaltet. Vorzugsweise weisen sowohl der erste als auch der dritte Werkzeugabschnitt Stirnflächen auf, die sich entlang einer zweiten Richtung (Y), welche senkrecht zu der axialen Richtung verläuft, erstrecken. Vorzugsweise liegen nach Verfahrensschritt b) diese Stirnflächen fest aneinander an. Vorzugsweise wird die erste Kavität beziehungsweise die zweite Öffnung des Formhohlraums des ersten Werkzeugabschnitts in axialer Richtung durch den dritten Werkzeugabschnitt abgeschlossen.

Nach einer weiteren bevorzugten Ausführungsform werden der erste, zweite, dritte und vierte Werkzeugabschnitt individuell erwärmt und gekühlt. Die Erwärmung erfolgt vorzugsweise mittels eines Lasers. Die Laserwellenlänge ist vorteilhaft daraufhin abgestimmt, dass ein möglichst großer Anteil der Laserenergie von dem Werkzeugmaterial absorbiert werden kann. Die Verwendung eines Lasers hat zum Vorteil, dass sehr präzise die Auswahl des zu erwärmenden Werkzeugabschnitts erfolgen kann. Es wären jedoch auch anderweitige Erwärmungsmethoden denkbar wie beispielsweise Heizdrähte oder Ähnliches. Vorteilhafterweise erfolgt die Kühlung der Werkzeugabschnitte mittels eines Kühlmittels. Vorzugsweise enthalten solche Kühlmittel Wasser und/oder Stickstoff und/oder CO₂.

Durch die Kühlung des ersten, zweiten und dritten Werkzeugabschnitts beziehungsweise des Spritzenkörpers in Verfahrensschritt d) und durch die damit einhergehende thermische Kontraktion des Materials folgt eine Volumenschwindung des Spritzenkörpers. Demzufolge entformt dieser vorteilhaft bereits aus dem Formhohlraum des ersten Werkzeugabschnitts. Der Spritzenkörper bleibt vorteilhafterweise jedoch auf dem ersten Spritzgusskern angeordnet und kann somit die weiteren Verfahrensschritte erfahren.

Nach einer weiteren bevorzugten Ausführungsform wird nach dem Verfahrensschritt f) der vierte Werkzeugabschnitt abgekühlt, wodurch das Verschlusselement abkühlt und durch die damit einhergehende Volumenschwindung aus dem Formhohlraum des vierten Werkzeugabschnitts entformt. Bevorzugt wird anschließend der Spritzenkörper mit dem integrierten Verschlusselement ausgeworfen und weiteren Prozessschritten zugeführt.

Nach einem weiteren Gedanken der Erfindung ist der erste Spritzgusskern teleskopartig ausgestaltet. Vorzugsweise ist der zweite zylindrische Abschnitt relativ zu dem ersten Abschnitt verlagerbar. Nach einer bevorzugten Ausführungsform weist der erste zylindrische Abschnitt eine durchgehende Bohrung auf in welcher ein weiteres zylindrisches Element, welches den zweiten zylindrischen Abschnitt umfasst, beweglich angeordnet ist. Dieses weitere zylindrische Element kann vorteilhaft mittels eines Antriebes in axialer Richtung (X) vor- und zurückverlagert werden. Bevorzugt befindet sich der zweite zylindrische Abschnitt in den Verfahrensschritten b) bis d) in einer maximalen axialen Position, so dass der zweite zylindrische Abschnitt den zweiten Spritzgusskern kontaktiert. Vorteilhafterweise wird zwischen Verfahrensschritt d) und e) der zweite Abschnitt in axialer Richtung von dem dritten Werkzeugabschnitt weg verlagert. Bevorzugt tritt dann in dem nachfolgenden Verfahrensschritt f) der zweite Kunststoff in den Kanal des Endstücks ein, wodurch eine Abdichtung des Kanals erzeugt wird. Dadurch, dass das erste und zweite Kunststoffmaterial keine stoffschlüssige Verbindung eingehen, ist die Abdichtung lösbar. Vorzugsweise wird vor Anwendung der Spritze die Abdichtung mit dem Verschlusselement von der Spritze entfernt.

Vorzugsweise sind das erste und das zweite Kunststoffmaterial unterschiedliche Materialien. Gemäß einer besonders bevorzugten Ausführungsform ist das erste Kunststoffmaterial ein Polymer-Kunststoff, bevorzugt ein Polyolefin, beispielsweise Polypropylen oder Polyethylen, besonders bevorzugt ein Cyclo-Olefines Polymer (COP) beziehungsweise ein Cyclo-Olefines Co-Polymer (COC). COC und COP sind im Gegensatz zu den teilkristallinen Polyolefinen wie Polyethylen und Polypropylen amorph und damit transparent. Derartige Kunststoffe zeichnen sich dadurch aus, dass sie eine hervorragende Biokompatibilität, insbesondere Blutverträglichkeit sowie eine äußerst geringe Wasseraufnahme/ Wasserdampfdurchlässigkeit aufweisen. Weiterhin zeigen diese Kunststoffe keine Reaktion mit den üblichen zur Anwendung kommenden Medikamenten.

Nach einer weiteren bevorzugten Ausführungsform ist das zweite Kunststoffmaterial ein thermoplastisches Elastomer.

Gemäß einer weiteren bevorzugten Ausführungsform sind das erste und das zweite Kunststoffmaterial die gleichen Materialien. Vorteilhafterweise ist dieses Material ein Polymer-Kunststoff, bevorzugt ein Polyolefin, beispielsweise Polypropylen oder Polyethylen, besonders bevorzugt ein Cyclo-Olefines Polymer (COP) beziehungsweise ein Cyclo-Olefines Co-Polymer (COC) sind. Wie bereits erwähnt eignen sich COC/COP besonders gut für vorfüllbare Spritzen aufgrund der hohen Barriereeigenschaften. Eine derartige Ausgestaltung ist für vorfüllbare Spritzen besonders vorteilhaft, da das Verschlussmaterial während der gesamten Lagerdauer der vorgefüllten Spritze in Kontakt mit dem Medium (Medikament/ Impfstoff) in der Spritze ist. Durch die oben genannten Eigenschaften der Materialen COP und COC wird ein möglichst geringer Einfluss des Verschlussmaterials auf das Medium gewährleistet.

Das einzuspritzende Kunststoffmaterial wird bevorzugt in einer Plastifiziereinheit plastifiziert. Eine solche Plastifiziereinheit umfasst unter anderem einen Plastifizierzylinder mit einer Schnecke und Heizelemente. Das plastifizierte Kunststoffmaterial wird dann vorzugsweise über einen Angusskanal der entsprechenden Einspritzdüse zugeführt.

In der Einspritzphase wird dabei das plastifizierte Kunststoffmaterial über die jeweilige Einspritzdüse unter einem hohen Druck in die Kavität eingespritzt. Der Druck liegt dabei bevorzugt zwischen einem Wert von ca. 1 MPa und ca. 200 MPa. Das plastifizierte Kunststoffmaterial hat bei dem Einspritzen vorzugsweise eine Temperatur von einigen 100°C. Die die Kavität bildenden Elemente haben jedoch eine geringere Temperatur. Bei Kontakt mit den vergleichsweise kalten Wandbereichen der Kavität erstarrt der Kunststoff bei Erreichen des Erstarrungspunkts des jeweiligen Materials. Um eine möglichst homogene Verteilung des Kunststoffmaterials in der Kavität zu gewährleisten, muss das Kunststoffmaterial entsprechend schnell in die Kavität eingebracht werden. Die Einspritzgeschwindigkeit liegt vorzugsweise bei einigen 10 ccm/s. COC und COP, weisen eine vergleichbar niedrige Viskosität auf und sind besonders gut dafür geeignet um fein strukturierte, dünnwandige Bauteile auch über lange Fließwege fehlerfrei herstellen zu können.

Nach einem vorteilhaften Gedanken der Erfindung werden die Kunststoffmaterialen jeweils über einen Heißkanal der entsprechenden Einspritzdüse zugeführt. Das von der Plastifiziereinheit zu der jeweiligen Einspritzdüse führende Angusssystem ist hierdurch vorteilhaft von dem Spritzgusswerkzeug thermisch isoliert. Bei anderweitigen Angusssystemen erstarrt nach dem Befüllen der Kavität das überbleibende Kunststoffmaterial in dem Angusssystem. Dieser sogenannte Anguss muss dann von dem Formteil abgetrennt werden. Dies erfordert zusätzliche Werkzeuge. Ferner muss das zusätzliche Kunststoffmaterial entsorgt werden. Bei der Verwendung eines Heißkanals wird das Angusssystem auf einer entsprechend hohen Temperatur gehalten, so dass der Anguss nicht erstarrt.

In einer bevorzugten Ausführungsform werden zwei, bevorzugter drei und besonders bevorzugt mehr als drei Aussparungen an dem Anschlusselement und dazu komplementäre Vorsprünge an dem Verschlusselement ausgebildet. Bei einer Anordung von zwei oder mehr Aussparungen bzw. Vorsprüngen sind diese bevorzugt in einer Umfangsrichtung in gleichmäßigen Winkelabständen zueinander versetzt. So wären beispielsweise zwei Vorsprünge bzw. Aussparungen um 180°, drei Vorsprünge bzw. Aussparungen um 120° und vier Vorsprünge bzw. Aussparungen um jeweils 90° zueinander versetzt.

In einer besonders bevorzugten Ausführungsform wird nach dem Verfahrensschritt d) in einem weiteren Verfahrensschritt d2) ein Dichtelement an dem vierten Werkzeugabschnitt angebracht, wobei der vierte Werkzeugabschnitt ein in den Formhohlraum des vierten Werkzeugabschnitts hineinragendes Halteelement aufweist, welches zum Halten des Dichtelements ausgestaltet ist. Weiterhin wird das Dichtelement bevorzugt in dem Verfahrensschritt e) an das hohlzylindrische Endstück des Spritzenkörpers gedrückt. Durch das Andrücken des Dichtelements an das hohlzylindrische Endstück des Spritzenkörpers wird das hohlzylindrische Endstück abgedichtet. Bevorzugt ist das Dichtelement aus einem Gummimaterial gefertigt. Allerdings kann das Dichtelement aus jedem anderen Material, wie beispielsweise Kunststoff, gefertigt sein, welches keine Reaktionen mit den üblicherweise verwendeten Medikamenten oder Stoffen hervorruft. Durch das Anordnen eines Dichtelements entsteht kein direkter Kontakt des zweiten Kunststoffmaterials mit dem Medikament, weswegen die Auswahl des zweiten Kunststoffmaterials nicht durch die Medikamentenkompatibilität eingeschränkt wird.

Bevorzugt wird das Dichtelement nach Beendigung des Verfahrensschrittes f) derart von dem zweiten Kunststoffmaterial umschlossen, so dass das Dichtelement nach dem Entformen des vierten Werkzeugabschnitts weiterhin an das hohlzylindrische Endstück des Spritzenkörpers gedrückt wird. Dies ist vorteilhaft, um ein Verrutschen des Dichtelements nach der Herstellung zu verhindern und eine zuverlässige Abdichtung der Spritze zu gewährleisten. Demnach ist das Dichtelement zumindest in Umfangsrichtung vollständig und in einer ersten axialen Richtung X zumindest teilweise von dem zweiten Kunststoffmaterial umhüllt.

Besonders bevorzugt wird zwischen den Verfahrensschritten d) und e) der zweite Spritzgusskern von dem Anschlusselement des Spritzenkörpers entformt und der dritte Werkzeugabschnitt von dem ersten Werkzeugabschnitt entfernt, wobei der zweite Spritzgusskern durch eine Drehbewegung des zweiten Spritzgusskerns von dem Innengewinde und der wenigstens einen Aussparung des Anschlusselements entformt wird. Eine Drehbewegung zum Entfernen des zweiten Spritzgusskerns ist vorteilhaft, um das ausgeformte Innengewinde sowie die wenigstens eine Aussparung nicht zu beschädigen. Dabei ist der zweite Spritzgusskern drehbar in dem vierten Werkzeugabschnitt gelagert.

Weiterhin werden alle Werkzeughälften nach Beendigung des Verfahrens voneinander über eine Relativbewegung zueinander und/oder zu dem Spritzenkörper entformt. Diese Bewegung kann sowohl eine rotatorische als auch eine translatorische Bewegung sein.

Vorzugsweise sind das erste und das zweite Kunststoffmaterial unterschiedliche Materialien. Gemäß einer besonders bevorzugten Ausführungsform ist das erste Kunststoffmaterial ein Polymer-Kunststoff, bevorzugt ein Polyolefin, beispielsweise Polypropylen oder Polyethylen, besonders bevorzugt ein Cyclo-Olefines Polymer (COP) beziehungsweise ein Cyclo-Olefines Co-Polymer (COC). COC und COP sind im Gegensatz zu den teilkristallinen Polyolefinen wie Polyethylen und Polypropylen amorph und damit transparent. Derartige Kunststoffe zeichnen sich dadurch aus, dass sie eine hervorragende Biokompatibilität, insbesondere Blutverträglichkeit sowie eine äußerst geringe Wasseraufnahme/Wasserdampfdurchlässigkeit aufweisen. Weiterhin zeigen diese Kunststoffe keine Reaktion mit den üblichen zur Anwendung kommenden Medikamenten. Diese Ausführungsform ist insbesondere vorteilhaft, falls ein zusätzliches Dichtelement in dem Verschlusselement vorgesehen ist, da auf diese Weise das zweite Kunststoffmaterial nicht in Kontakt mit dem Medikament gelangt und demnach lediglich das Dichtelement die besonderen Anforderungen erfüllen muss.

Nach einer weiteren bevorzugten Ausführungsform ist das zweite Kunststoffmaterial ein thermoplastisches Elastomer.

Gemäß einer weiteren bevorzugten Ausführungsform sind das erste und das zweite Kunststoffmaterial die gleichen Materialien. Vorteilhafterweise ist dieses Material ein Polymer-Kunststoff, bevorzugt ein Polyolefin, beispielsweise Polypropylen oder Polyethylen, besonders bevorzugt ein Cyclo-Olefines Polymer (COP) beziehungsweise ein Cyclo-Olefines Co-Polymer (COC) sind. Wie bereits erwähnt eignen sich COC/COP besonders gut für vorfüllbare Spritzen aufgrund der hohen Barriereeigenschaften. Eine derartige Ausgestaltung ist für vorfüllbare Spritzen besonders vorteilhaft, da das Verschlussmaterial während der gesamten Lagerdauer der vorgefüllten Spritze in Kontakt mit dem Medium (Medikament/ Impfstoff) in der Spritze ist. Durch die oben genannten Eigenschaften der Materialen COP und COC wird ein möglichst geringer Einfluss des Verschlussmaterials auf das Medium gewährleistet. Diese Ausführungsform ist insbesondere ohne die Verwendung eines zusätzlichen Dichtelements relevant, da dort das zweite Kunststoffmaterial einen direkten Kontakt mit dem Medikament aufweist.

Die Aufgabe der Erfindung wird ebenso durch eine Spritze nach Anspruch 14 mit einem daran angeformten Verschlusselement sowie mit wenigstens einem an dem Verschlusselement angeordneten Vorsprung gelöst, welche nach einem Verfahren gemäß einer der vorhergehenden Ausführungsformen hergestellt ist.

In einer bevorzugten Ausführungsform der Spritze ist an dem Verschlusselement ein Dichtelement angeordnet.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig. 1: eine Schnittdarstellung des Spritzgusswerkzeugs
- Fig. 2: eine weitere Schnittdarstellung des Spritzgusswerkzeugs;
- Fig. 3: eine weitere Schnittdarstellung des Spritzgusswerkzeugs;
- Fig. 4: eine Schnittdarstellung des Spritzgusswerkzeugs gemäß einer weiteren Ausführungsform;
- Fig. 5: eine Schnittdarstellung des Spritzgusswerkzeugs gemäß einer weiteren Ausführungsform;
- Fig. 6: eine Schnittdarstellung eines Spritzenkörpers;
- Fig. 7: eine Schnittdarstellung eines Spritzenkörpers gemäß einer weiteren Ausführungsform;
- Fig. 8: eine Schnittdarstellung eines Spritzenkörpers gemäß einer weiteren Ausführungsform;
- Fig. 9: eine Seitenansicht des Verschlusselements;
- Fig. 10: eine Schnittdarstellung des Spritzgusswerkzeugs;
- Fig. 11: eine weitere Schnittdarstellung des Spritzgusswerkzeugs;
- Fig. 12: eine weitere Schnittdarstellung des Spritzgusswerkzeugs;
- Fig. 13: eine Schnittdarstellung des Spritzgusswerkzeugs einer bevorzugten Ausführungsform;
- Fig. 14: eine weitere Schnittdarstellung des Spritzgusswerkzeugs der bevorzugten Ausführungsform gemäß Fig. 3;
- Fig. 15: eine weitere Schnittdarstellung des Spritzgusswerkzeugs der bevorzugten Ausführungsform gemäß Fig. 3;
- Fig. 16: eine Schnittdarstellung des vierten Werkzeugabschnitts;
- Fig. 17: eine Schnittdarstellung einer Spritze der bevorzugten Ausführungsform gemäß Fig. 3;
- Fig. 18: einen vergrößerten Ausschnitt des distalen Endes des Spritzenkörpers aus Fig. 3;
- Fig. 19: einen vergrößerten Ausschnitt des distalen Endes mit Verschlusselement gemäß Fig. 5.

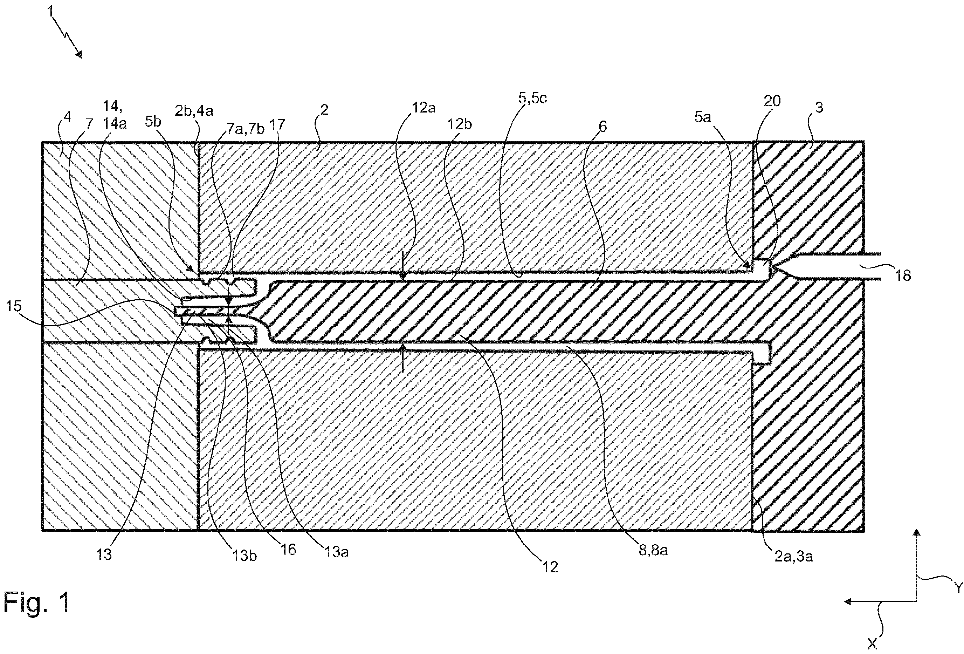

In den Figuren 1 bis 3 ist das Spritzgusswerkzeug prinzipiell dargestellt in verschiedenen Phasen des erfindungsgemäßen Spritzgießverfahrens zum Herstellen eine Spritze (100) mit einem integrierten Verschlusselement (101).

In Verfahrensschritt a) wird das Spritzgusswerkzeug (1) bereitgestellt, welches einen ersten (2), einen zweiten (3) und einen dritten Werkzeugabschnitt (4) umfasst, wobei der erste Werkzeugabschnitt (2) einen sich entlang einer axialen Richtung (X) erstreckenden beidseitig offenen Formhohlraum (5) aufweist und wobei der zweite Werkzeugabschnitt (3) einen ersten Spritzgusskern (6) und der dritte Werkzeugabschnitt (4) einen zweiten Spritzgusskern (7) aufweist.

In Verfahrensschritt b) wird das Spritzgusswerkzeug (1) geschlossen, so dass der erste Werkzeugabschnitt (2) den zweiten (3) und dritten Werkzeugabschnitt (4) kontaktiert und der erste (6) und zweite Spritzgusskern (7) durch jeweils eine Öffnung (5a, 5b) in den Formhohlraum (5) des ersten Werkzeugabschnitts (2) in diesen eintreten und sich schließlich kontaktieren, wodurch diese Werkzeugabschnitte (2, 3, 4) eine erste Kavität (8) bilden.

In Verfahrensschritt c) wird ein erstes Kunststoffmaterial in die erste Kavität (8) eingespritzt, wodurch ein hohlzylindrischer Spritzenkörper (102), an dessen distalen Ende (102a) ein Endbereich (103) angeordnet ist, ausgebildet wird, wobei der Endbereich (103) ein mit einem Innengewinde (104) versehenes Anschlusselement (105) und ein hohlzylindrisches Endstück (106), welches von dem Anschlusselement (105) zumindest abschnittsweise umrandet ist, aufweist.

In Verfahrensschritt d) werden die Werkzeugabschnitte (2, 3, 4) abgekühlt, wodurch der Spritzenkörper (102) abkühlt und aushärtet.

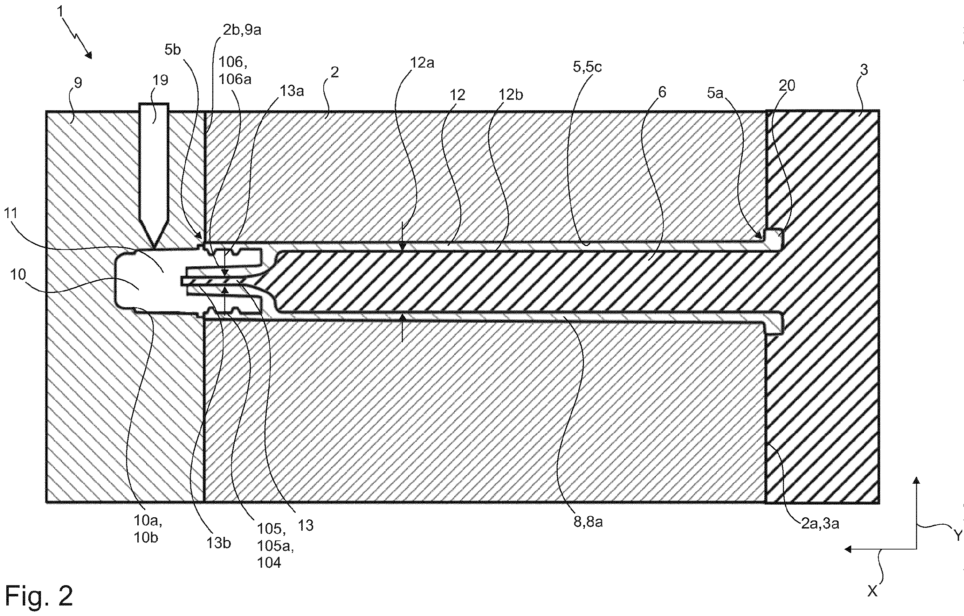

In Verfahrensschritt e) wird der erste Werkzeugabschnitt (2) mit einem einseitig geschlossenen Formhohlraum (9) versehenen vierten Werkzeugabschnitt kontaktiert, wodurch eine zweite Kavität (11) an dem distalen Ende (102a) des Spritzenkörpers (102) gebildet wird. Dies ist in Fig. 2 dargestellt.

In Verfahrensschritt f) wird ein zweites Kunststoffmaterial in die zweite Kavität (11) eingespritzt, wodurch das Verschlusselement (101) an das Anschlusselement (105) angeformt wird, wobei das erste und das zweite Kunststoffmaterial keine stoffschlüssige Verbindung eingehen.

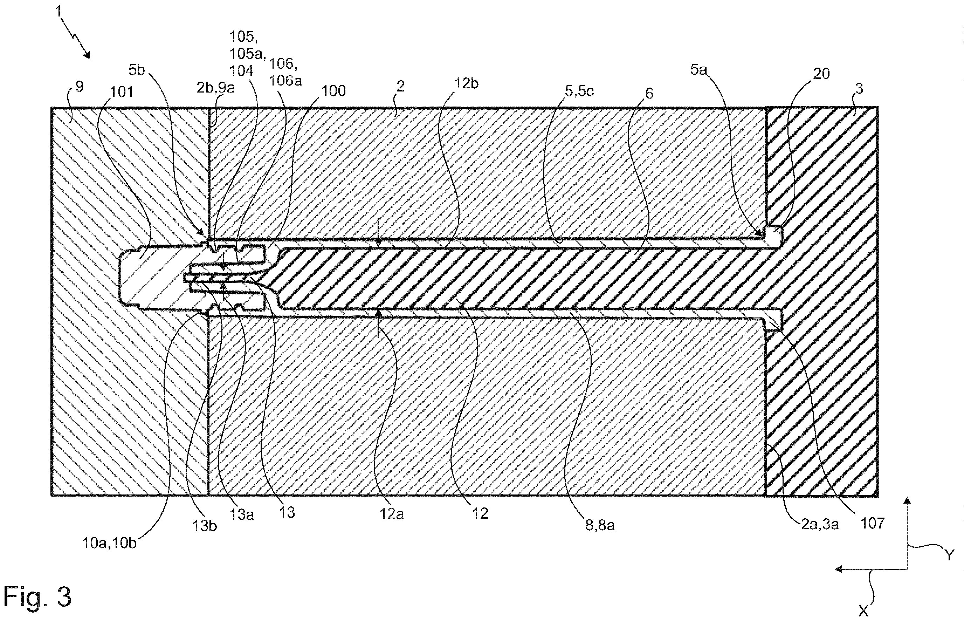

In Fig. 3 ist schließlich die Spritze (100) mit dem integrierten Verschlusselement (101) in dem Spritzgusswerkzeug dargestellt. Das Verschlusselement (101) beziehungsweise der Spritzenkörper (102) werden weiter abgekühlt und härten somit aus. Anschließend wird der vierte Werkzeugabschnitt entfernt und die Spritze (100) mit dem integrierten Verschlusselement (101) kann ausgeworfen werden.

Der Formhohlraum (5) des ersten Werkzeugabschnitts (2) weist eine Längserstreckung entlang der axialen Richtung (X) und eine Höhenerstreckung entlang einer zweiten Richtung (Y) auf. Ferner ist dieser Formhohlraum (5) hohlzylindrisch mit einer kreisförmigen Grundfläche ausgebildet. Darüber hinaus weist der Formhohlraum (5) des ersten Werkzeugabschnitts (2) eine erste Öffnung (5a), in welche der erste Spritzgusskern (6) in Verfahrensschritt b) eintritt und eine zweite Öffnung (5b), in welche der zweite Spritzgusskern (6) in Verfahrensschritt b) eintritt, auf.

Der erste (6) und zweite Spritzgusskern (7) weisen ebenso eine Längserstreckung entlang der axialen Richtung (X) auf. Ferner sind diese zylindrisch ausgestaltet. Der erste Spritzgusskern (6) umfasst einen ersten zylindrischen Abschnitt (12) mit einem ersten Durchmesser (12a) und einen zweiten zylindrischen Abschnitt (13) mit einem zweiten kleineren Durchmesser (13a). Dabei sind der erste (12) und der zweite zylindrische Abschnitt (13) in axialen Richtung (X) angrenzend.

Eine innere Wandung (5c) des Formhohlraums (5) des ersten Werkzeugabschnitts und eine äußere Wandung (12b) des ersten zylindrischen Abschnitts (12) des ersten Spritzgusskerns (6) einen ersten Abschnitt (8a) der ersten Kavität (8), mittels welchem der hohlzylindrischen Spritzenkörper (102) gebildet wird.

Der zweite Spritzgusskern (7) weist einen in axialer Richtung (X) einseitig offenen Formholraum (14) und eine Ausnehmung (15) an einer den Formholraum (14) in axialer Richtung (X) begrenzenden Innenwand (14a) auf.

In Verfahrensschritt b) wird der zweite zylindrische Abschnitt (13) des ersten Spritzgusskerns in der Ausnehmung (15) des zweiten Spritzgusskerns (7) teilweise aufgenommen. Eine äußere Wandung (13b) des zweiten zylindrischen Abschnitts (13) und eine innere Wandung (14a) des Formholraums (14) des zweiten Spritzgusskerns (7) bilden die dritte Kavität (16), mittels welcher das konische Endstück (106) ausgebildet wird. Durch die teilweise Aufnahme des zweiten zylindrischen Abschnitts (13) in der Ausnehmung (15) des Formhohlraums (14) ragt der zweite zylindrische Abschnitt (13) über die Innenwand (14a) hinaus. Durch diese Ausgestaltung wird gewährleistet, dass in den Kanal (108) des Endstücks (106) kein erstes Kunststoffmaterial eintritt und diesen somit verschließt.

Das mit dem Innengewinde (104) versehene Anschlusselement (101) wird mittels einer vierten Kavität (17) gebildet. Diese vierte Kavität (17) wird durch Außenbereich (7a) des zweiten Spritzgusskerns (7) und der inneren Wandung (5c) des Formhohlraums (5) des ersten Werkzeugabschnitts (2) gebildet. Dieser Außenbereich (7a) des zweiten Spritzgusskerns (7) weist Ausnehmungen und Vorsprünge (7b) auf, durch welche das Innengewinde (104) an dem Anschlusselement (101) ausgebildet wird.

Der den ersten Spritzgusskern (7) aufweisende zweite Werkzeugabschnitt (3) ist abschnittsweise T-förmig ausgestaltet. Der erste (2) als auch der zweite Werkzeugabschnitt (3) weisen Stirnflächen (2a, 3a) auf, die sich entlang der zweiten Richtung (Y) erstrecken. Diese Stirnflächen (2a, 3a) liegen nach Verfahrensschritt b) fest aneinander an. Die erste Kavität (8), beziehungsweise die erste Öffnung (5a) des Formhohlraums (5) werden somit in axialer Richtung (X) durch den zweiten Werkzeugabschnitt (3) abgeschlossen. Der zweite Werkzeugabschnitt (3) weist weiterhin eine Ausnehmung (20) auf, welche zur ersten Kavität (8) hin offen ist und mittels welcher eine Fingerauflage (107) am proximalen Ende (102b) des Spritzenkörpers (102) gebildet wird.

Das erste Kunststoffmaterial wird dabei durch eine erste Einspritzdüse (18), welche in dem zweiten Werkzeugabschnitt (3) angeordnet ist, eingespritzt. Diese erste Einspritzdüse (18) ist mit der Ausnehmung (20) verbunden. Das erste Kunststoffmaterial wird also über diese Ausnehmung (20) in die erste Kavität (8) eingespritzt.

Der den zweiten Spritzgusskern (7) aufweisende dritte Werkzeugabschnitt (4) ist ebenso abschnittsweise T-förmig ausgestaltet. Der erste (2) als auch der dritte Werkzeugabschnitt (4) weisen Stirnflächen (2b, 4a) auf, die sich entlang einer zweiten Richtung (Y) erstrecken. Nach Verfahrensschritt b) liegen diese Stirnflächen (2b, 4a) fest aneinander an und die erste Kavität (8) beziehungsweise die zweite Öffnung (5b) des Formhohlraums (5) werden somit in axialer Richtung durch den dritten Werkzeugabschnitt (4) abgeschlossen. Der zweite Spritzgusskern (7) ist dabei drehbar in einer Bohrung des dritten Werkzeugabschnitts (4) angeordnet. Der zweite Spritzgusskern (7) kann somit zwischen den Verfahrensschritten d) und e) durch eine Drehbewegung von dem Anschlusselement (105) beziehungsweise von dem Innengewinde (104) des Spritzenkörpers (102) entformt werden.

Nach dem Entformen des zweiten Spritzgusskerns (7) und dem Entfernen des dritten Werkzeugabschnitts (4) kontaktiert der erste Werkzeugabschnitt (2) den vierten Werkzeugabschnitt (9). Die Stirnflächen (2b) des ersten Werkzeugabschnitts (2) legen also an entsprechenden Stirnflächen (9a) des vierten Werkzeugabschnitts (9) fest an. Wie in Fig. 2 erkennbar ragt das in Verfahrensschritt c) gefertigte Endstück (106) über die Stirnflächen (2b) des ersten Werkzeugabschnitts (2) hinaus und in den Formhohlraum (14) des vierten Werkzeugabschnitts (9) hinein.

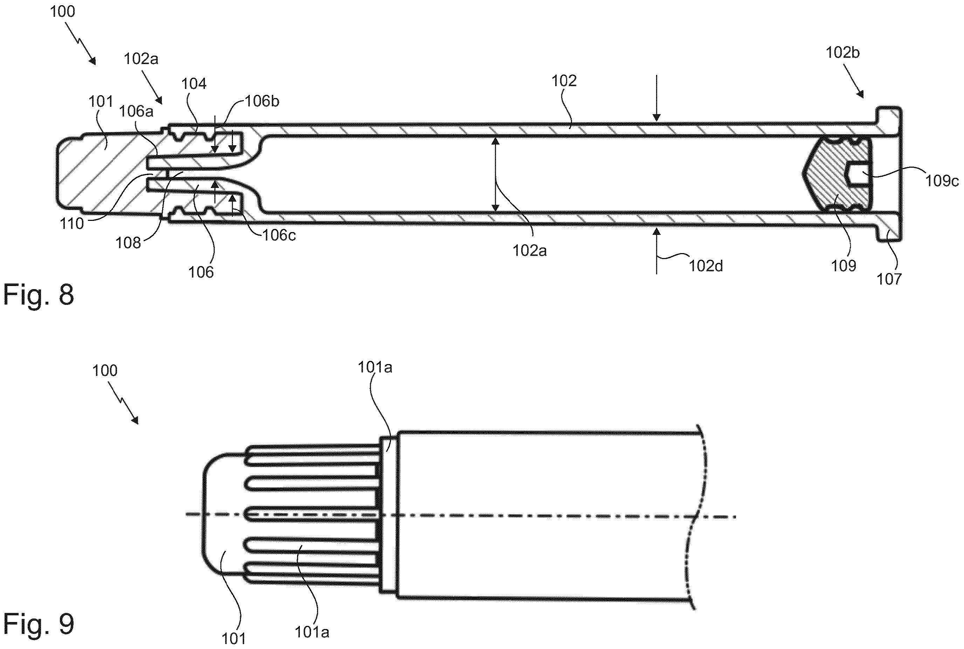

Die in Verfahrensschritt e) gebildete zweite Kavität (11) wird durch eine innere Wandung (14a) des Formhohlraums (14) des vierten Werkzeugabschnitts (9) und durch Außenbereiche des Endbereichs (103) des Spritzenkörpers (102) gebildet. Diese Außenbereiche sind die äußere Wandung (106a) des Endstücks (106) und die innere Wandung (105a) des Anschlusselements (105) beziehungsweise das Innengewinde (104) des Anschlusselements (105). An der inneren Wandung (10a) des Formhohlraums (10) des vierten Werkzeugabschnitts (9) sind Ausnehmungen und/oder Vorsprünge (10b) angeordnet, so dass das Verschlusselement (101) mit entsprechenden Vorsprüngen (101a), beispielsweise Längsrippen, gebildet wird. Solche Vorsprünge sind in Fig. 9 dargestellt.

Das zweite Kunststoffmaterial wird durch eine zweite Einspritzdüse (19) in die zweite Kavität (11) eingespritzt. Diese zweite Einspritzdüse (19) ist in dem vierten Werkzeugabschnitt (9) angeordnet und mit dem Formhohlraum (10) des vierten Werkzeugabschnitts (9) verbunden.

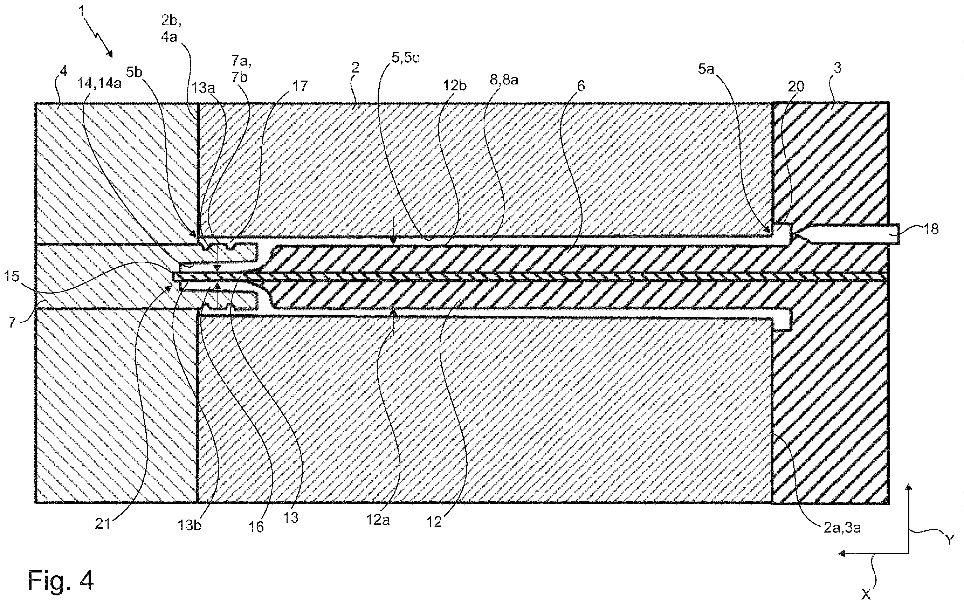

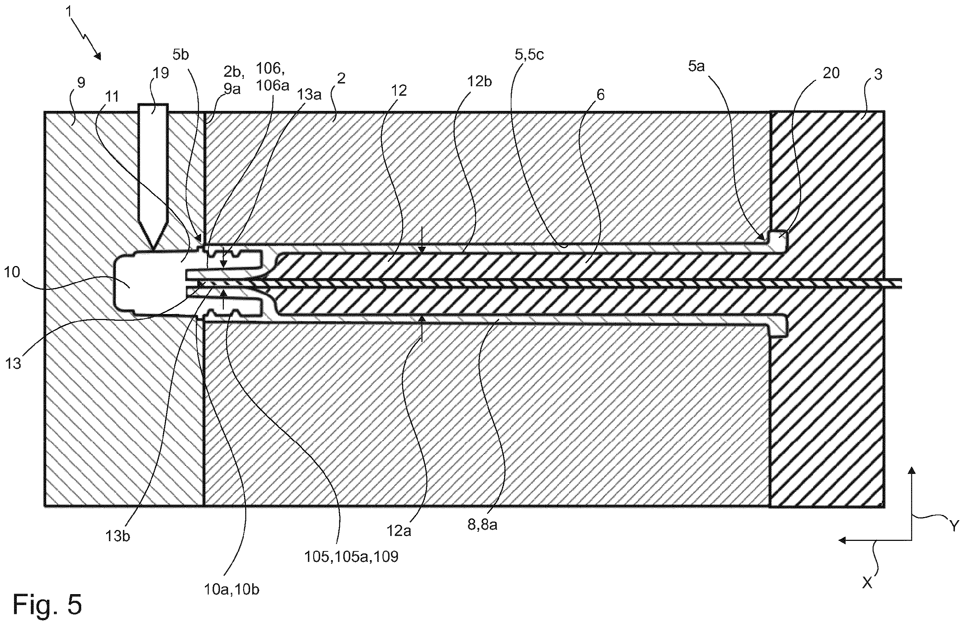

In den Fig. 4 und 5 ist ein Spritzgusswerkzeug gemäß einer weiteren Ausführungsform dargestellt. Hierbei ist der erste Spritzgusskern (6) teleskopartig ausgestaltet, wobei der zweite zylindrische Abschnitt (13) relativ zu dem ersten Abschnitt (12) verlagerbar ist.

In den Verfahrensschritten b) bis d) befindet sich dabei der zweite zylindrische Abschnitt (13) in einer maximalen axialen Position (21), so dass der zweite zylindrische Abschnitt (13) den zweiten Spritzgusskern (7) kontaktiert. Zwischen Verfahrensschritt d) und e) wird nun jedoch der zweite zylindrische Abschnitt (13) in axialer Richtung (X) von dem dritten Werkzeugabschnitt (4) weg verlagert. Es wird somit ein Abschnitt des inneren Kanals (108) des Endstücks (106) freigegeben, so dass in dem nachfolgenden Verfahrensschritt f) der zweite Kunststoff in den Kanal (108) des Endstücks (106) eintritt. Dieser eingetretene Kunststoff stellt eine Abdichtung des Kanals (108) beziehungsweise einen Dichtabschnitt (110) des Verschlusselements (101) dar. Eine entsprechend hergestellte Spritze (100) ist in Fig. 8 dargestellt.

Die teleskopartige Ausgestaltung wird dadurch realisiert, dass ein zylindrisches stabförmiges Element in einer zentrisch angeordneten Durchgangsbohrung in dem ersten Spritzgusskern (6) angeordnet ist. Dieses zylindrische stabförmige Element umfasst den zweiten Zylindrischen Abschnitt (13) des ersten Spritzgusskerns (6) und kann relativ zu dem ersten zylindrischen Abschnitt in axialer Richtung (X) verlagert werden. Dies kann beispielsweise durch einen Antrieb erfolgen.

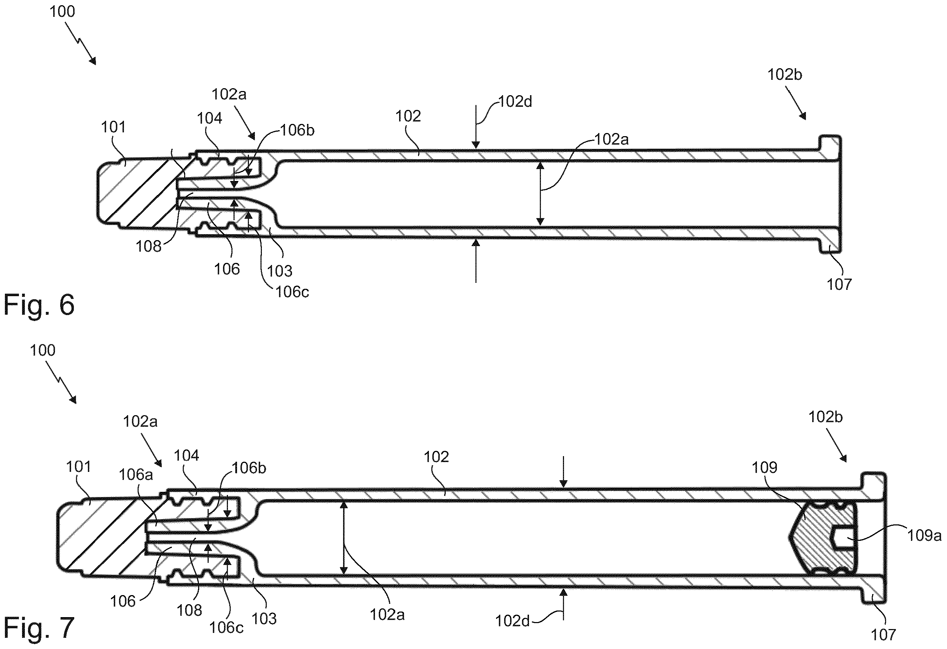

In den Figuren 6 bis 9 sind entsprechende Ausführungsformen von Spritzen (100) mit einem integrierten Verschlusselement (101) dargestellt.

Die dargestellten vorfüllbaren Spritzen (100) sind für medizinische Anwendungen geeignet und umfassen einen hohlzylindrischen Spritzenkörper (102) an dessen distalen Ende (102a) ein Endbereich (103) angeordnet ist. Der Endbereich (103) umfasst ein mit einem Innengewinde (104) versehenes Anschlusselement (105) und ein hohlzylindrisches Endstück (106), welches von dem Anschlusselement (105) zumindest abschnittsweise umrandet ist.

Das Endstück (106) weist einen inneren Kanal auf, welcher einen kleineren Innendurchmesser (106b) als der Innendurchmesser (102c) des Spritzenkörpers (102) aufweist. Ferner ist der Außendurchmesser (102d) des Spritzenkörpers (102) größer als der Außendurchmesser (106c) des Endstücks (106). Das Endstück (106) ist in dieser Ausführungsform als ein Konus, dessen Außendurchmesser (106b) sich kontinuierlich verjüngt ausgebildet. Bevorzugt ist dabei eine flache Steigung zwischen 4% und 8%. Besonders bevorzugt ist der Konus gemäß der ISO-Norm 594 mit einer Steigung von 6% ausgebildet und stellt somit einen männlichen Luer-Konus dar. Es sind aber auch anderweitige Formen des Endstücks denkbar.

Das Endstück (106) ist dabei zentrisch von dem Anschlusselement (105) umgeben. Ferner ist die Länge des Endstücks (106) entlang der Längsachse X₁ größer als die Länge des Anschlusselements (105) entlang der Längsachse X₁, so dass das Endstück (106) über das Anschlusselement (105) hervorstehend ist. Durch eine derartige Anordnung kann eine Verbindung zwischen der Spritze (100) und beispielsweise einem Transfersystem (hier nicht dargestellt) verriegelt und gegen ein versehentliches Lösen gesichert werden. Vorzugsweise ist das Endstück und das Anschlusselement (105) gemäß ISO-Norm 594 ausgestaltet, wodurch ein Luer-Lock System realisiert ist.

In den Fig. 7 und 8 ist die Spritze (100) mit einem Kolben (109) versehen, Dabei ist in dem Hohlraum des Spritzenkörpers (102) ein Kolben (109), auch Kolbenstopfen oder Stopfen genannt, entlang einer Längsrichtung (X₁) beweglich angeordnet. Dabei besteht zumindest die Mantelfläche des Kolbens (109) aus einem elastischen Material, so dass der Kolben dichtend an einer Innenwand des hohlzylindrischen Spritzenkörpers anliegt. Der Kolben (109) weist an seinem proximalen Ende eine Gewinde-Sackbohrung (109a) auf, in welche im Applikationsfall eine Kolbenstange mit einem kopfseitigen Gewinde einschraubbar ist. Alternativ sind anstatt einer Schraubverbindung auch anderweitige Verbindungsarten, wie beispielsweise Steckverbindungen, denkbar. Darüber hinaus ist der Kolben (109) an seinem distalen Ende in distaler Richtung (X₁) konisch verjüngend bzw. spitz zulaufend ausgebildet. Gerade bei vorgefüllten Spritzen ist es besonders wichtig, dass der Kolben ausreichend dichtend an der Innenwand des Spritzenkörpers (102) anliegt, da ein unerwünschtes Austreten des sich in dem Hohlraum befindlichen Mediums (Medikament) verhindert werden soll. Ebenso ist ein Eintreten von Verunreinigungen in die Spritze unerwünscht. Gleichzeitig muss aber auch ein bequemes leichtgängiges Verschieben des Kolbens (109) möglich sein, um das Medium durch den Kanal des Endstücks (106) aus der Spritze (100) hinauszubefördern. Hierzu werden üblicherweise die Innenwände des Spritzenkörpers (102) beschichtet bzw. silikonisiert. Das elastische Material des Kolbens muss weiterhin verträglich gegenüber der zu wählenden Sterilisationsart sein.

Derartige vorfüllbare Spritzen(-körper) werden vom Primärpackmittelhersteller unter Reinraumbedingungen hergestellt, abgepackt, sterilisiert und abfüllfertig direkt in den Reinraum des Abfüllers, beispielsweise ein Pharmazeut, geliefert und können ohne weitere Behandlungsschritte zur Abfüllung eingesetzt werden. Die vorfüllbaren Spritzen werden durch den Abfüller von der proximalen Seite bzw. der Flanschseite befüllt. Um dies zu ermöglichen, muss das distale Ende der Spritze (100) bzw. des Endstücks (106) mittels des Verschlusselements (101) vorher verschlossen sein.

Nachdem der Spritzenkörper (102) mit dem erfindungsgemäßen Verschlusselement (101) von dem Abfüller seitens des proximalen Endes bzw. der Flanschseite mit dem Medium befüllt worden ist, wird der Kolben (109) eingesetzt. Der Kolben (109) wird dabei durch einen Greifer in radialer Richtung komprimiert und seitens der Flanschseite in den befüllten Spritzenkörper (102) eingeführt.

In Figur 8 ist eine Ausführungsform einer Spritze (100) mit einem integrierten Verschlusselement (101) dargestellt, wobei das Verschlusselement (101) einen Dichtabschnitt (110) aufweist. Dieser Dichtabschnitt (110) ragt in den inneren Kanal (108) des Endstücks (106) und dichtet diesen Kanal (108) somit ab. Dadurch dass das erste Kunststoffmaterial des Spritzenkörpers (102) und das zweite Kunststoffmaterial des Verschlusselements (101) keine stoffschlüssige Verbindung eingehen ist der Dichtabschnitt (110) mit dem Verschlusselements (101) aus dem Kanal (108) beziehungsweise von dem Spritzenkörper (102) lösbar.

In Fig. 9 ist eine Seitenansicht des an dem Spritzenkörper (102) angeordneten Verschlusselements (101) dargestellt. Das Verschlusselement (101) ist kreiszylindrisch ausgebildet und weist an seiner Oberfläche Längsrippen und eine radial umlaufende Rippe auf.

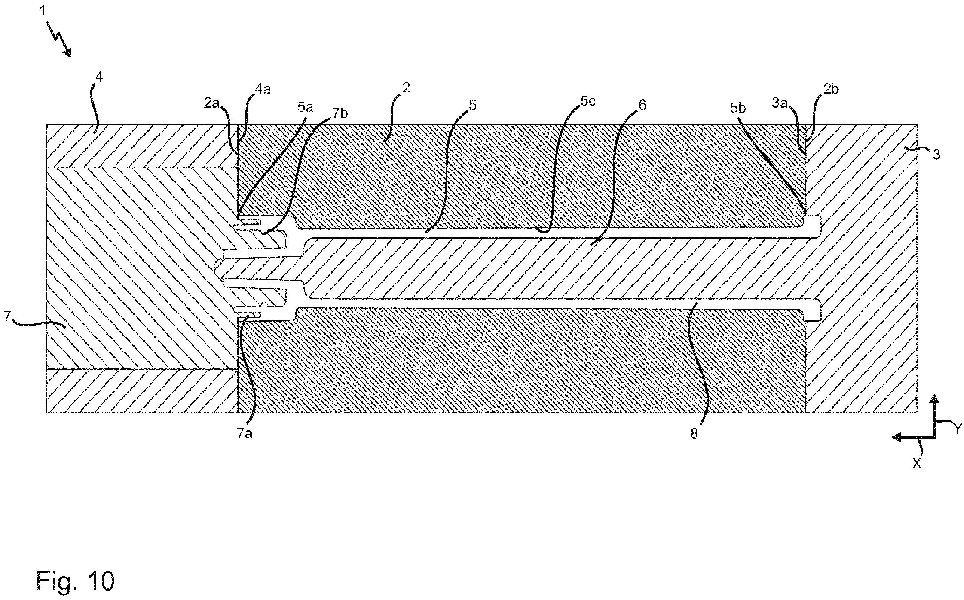

In den Figuren 10 bis 15 ist das Spritzgusswerkzeug prinzipiell dargestellt in verschiedenen Phasen des erfindungsgemäßen Spritzgießverfahrens zum Herstellen eine Spritze (100) mit einem integrierten Verschlusselement (101) sowie an dem Verschlusselement angeordneten Vorsprüngen (111).

Figur 10 zeigt dabei ein Spritzgusswerkzeug (1), welches einen ersten (2), einen zweiten (3) und einen dritten Werkzeugabschnitt (4) umfasst. Dabei weist der erste Werkzeugabschnitt (2) einen in der ersten axialen Richtung (X) beidseitig geöffneten Formhohlraum (5) auf. Der zweite Werkzeugabschnitt (3) weist dabei einen ersten Spritzgusskern (6) auf, welcher durch eine Öffnung (5b) in den Formhohlraum (5) eindringt. Der Spritzgusskern (6) erstreckt sich dabei in der ersten axialen Richtung (X) durch den gesamten Formhohlraum (5) und ist rotationssymmetrisch in Form einer Spritze ausgebildet. Der dritte Werkzeugabschnitt (4) weist ebenfalls einen Spritzgusskern (7) auf. Dieser Spritzgusskern (7) dringt durch eine Öffnung (5b) ebenfalls in den Formhohlraum (5) ein. Der Spritzgusskern (7) weist dabei unter anderem Vorsprünge (7a) und Vertiefungen (7b) auf. Die Vorsprünge (7a) dienen dazu, Aussparungen (112) an dem Anschlusselement (105) auszubilden. Durch die Vertiefungen (7b) an dem Spritzgusskern (7) wird an dem Anschlusselement (105) ein Innengewinde ausgebildet.

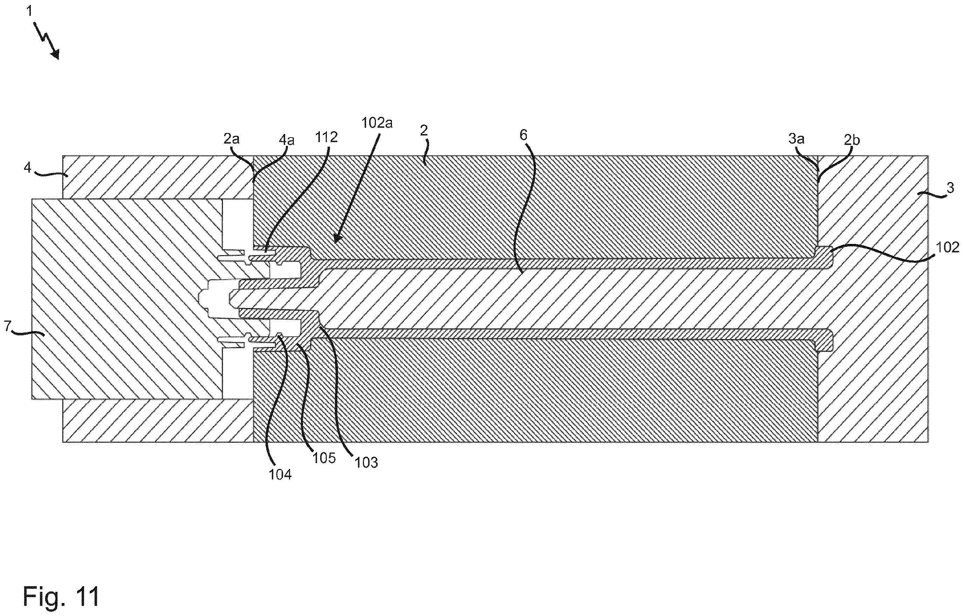

Figur 11 zeigt demnach ein Spritzgusswerkzeug (1) gemäß Verfahrensschritt b), da sich die Werkzeugabschnitte (2, 3, 4) sowie die Spritzgusskerne (6, 7) bereits kontaktieren. In diesem Fall kontaktieren sich eine erste Stirnfläche (2a) des ersten Werkzeugabschnitts (2) und eine Stirnfläche (4a) des dritten Werkzeugabschnitts (4) sowie die zweite Stirnfläche (2b) des ersten Werkzeugabschnitts (2) und eine Stirnfläche (3a) des zweiten Werkzeugabschnitts (3). Durch das Kontaktieren der verschiedenen Elemente wird eine erste Kavität (8) gebildet.

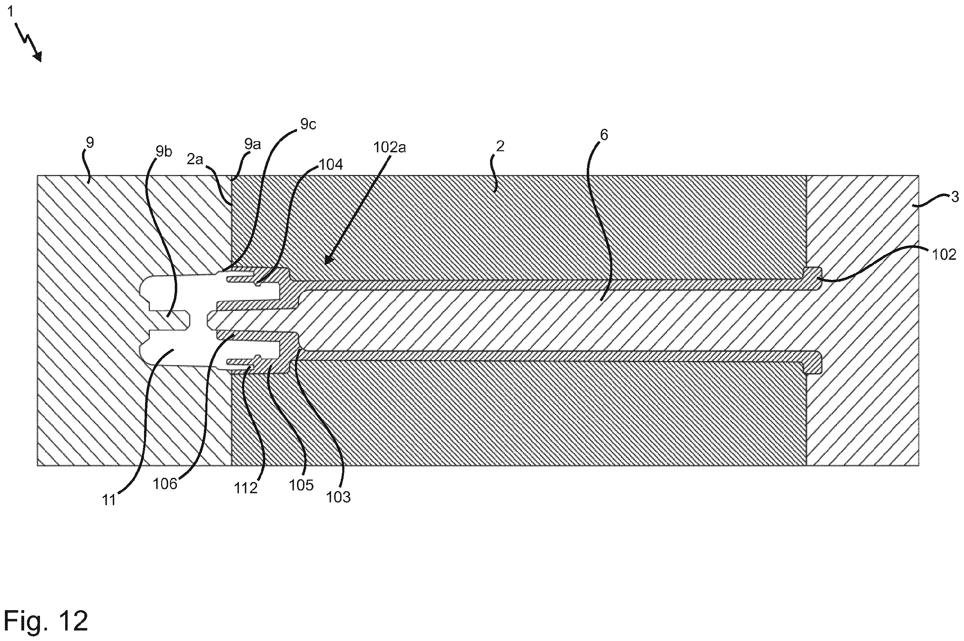

Figur 12 zeigt ein Spritzgusswerkzeug (1) nach Beendigung der Verfahrensschritte c) und d). In dieser Darstellung ist bereits ein hohlzylindrischer Spritzenkörper (102) ausgebildet, welcher an seinem distalen Ende (102a) einen Endbereich (103) aufweist, welcher ein Anschlusselement (105) umfasst. Dieses Anschlusselement (105) weist dabei ein Innengewinde (104) sowie eine Aussparung (112) auf. Das heißt, dass die erste Kavität (8) bereits vollständig mit dem ersten Kunststoffmaterial ausgefüllt ist und der geformte Spritzenkörper (102) auf einer Seite die innere Wandung (5c) des Formhohlraums (5) berührt, wobei auf der gegenüberliegenden Seite der Spritzgusskern (6) kontaktiert wird. Wie in Figur 2 weiter dargestellt, wird der Spritzgusskern (7) des dritten Werkzeugabschnitts (4) von dem Spritzenkörper (102) entformt, wobei der Spritzgusskern (7) dabei eine Drehbewegung ausführen muss, um von dem ausgebildeten Innengewinde (104) und den Aussparungen (112) gelöst zu werden, ohne diese Teile zu beschädigen.

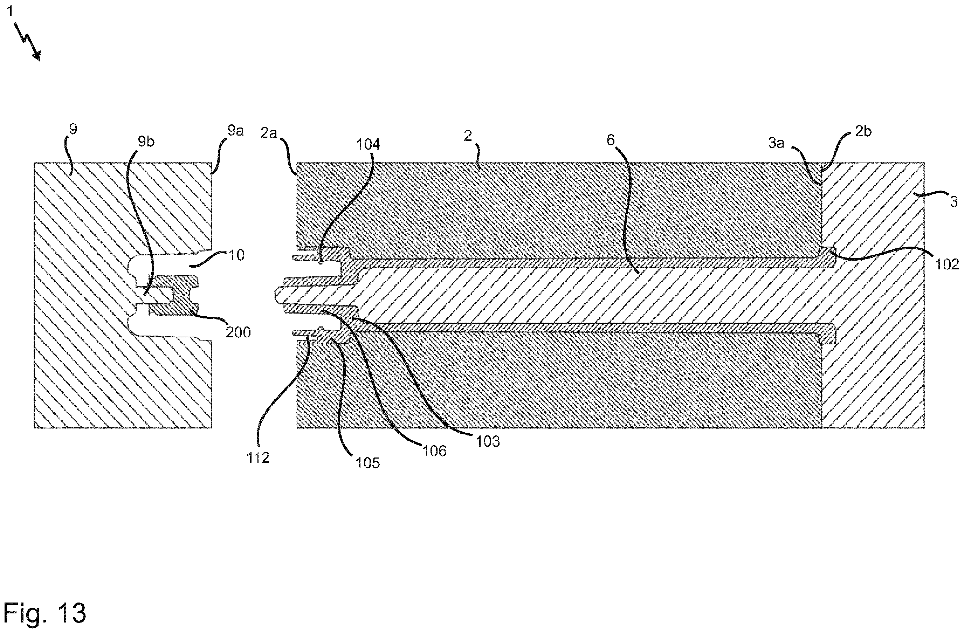

Figur 13 zeigt eine weitere Schnittdarstellung des Spritzgusswerkzeugs (1). Hierbei wurde ein vierter Werkzeugabschnitt (9) bereits mit dem ersten Werkzeugabschnitt (2) in Kontakt gebracht. Dabei kontaktieren sich die Stirnfläche (9a) des vierten Werkzeugabschnitts (9) und die erste Stirnfläche (2a) des ersten Werkzeugabschnitts (2) und bilden eine zweite Kavität (11). Die Kavität (11) wird dabei unter anderem durch den Formholraum (10) des vierten Werkzeugabschnitts (9) sowie die Aussparungen (112) des Anschlusselements (105) gebildet. In dieser Darstellung ist der Spritzenkörper (102) bereits ausgeformt und weist an seinem distalen Ende (102a) einen Endbereich (103) mit einem Anschlusselement (105) auf, wobei das Anschlusselement (105) ein Innengewinde (104) und Aussparungen (112) umfasst. Dieser vierte Werkzeugabschnitt (9) weist zudem ein Halteelement (9b) auf. Beim Einspritzen des zweiten Kunststoffmaterials in die zweite Kavität (11) wird nun ein Verschluss (101) an dem Anschlusselement (105) angeformt, wobei in dieser Ausführungsform das hohlzylindrische Endstück (106) durch das zweite Kunststoffmaterial verschlossen wird.

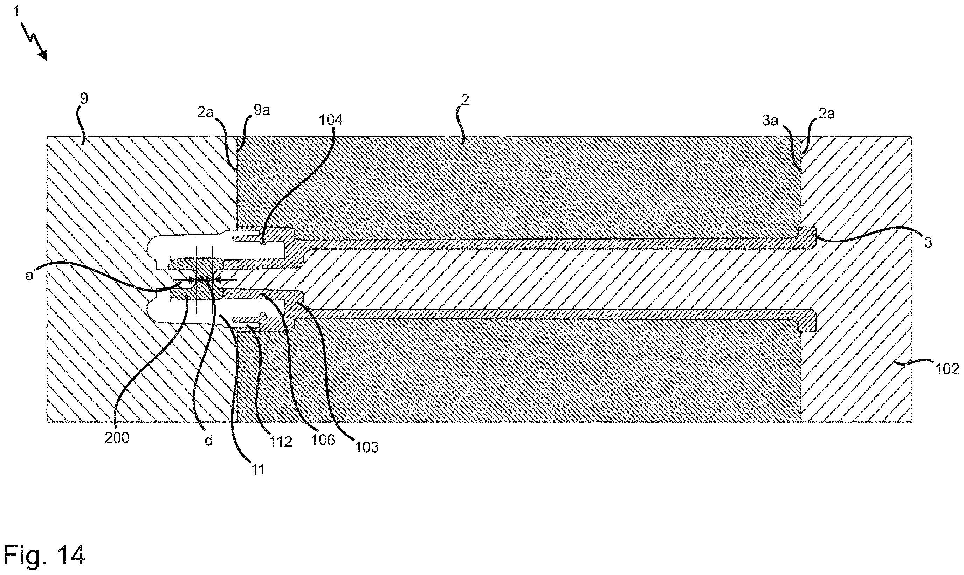

Figur 14 zeigt eine Schnittdarstellung des Spritzgusswerkzeugs (1) zur Herstellung einer Spritze (100) gemäß einer bevorzugten Ausführungsform. In dieser Darstellung ist der Spritzenkörper (102), wie auch in Figur 3, nach Beendigung der Verfahrensschritte a) - d) bereits ausgeformt. Nun wird wie in Figur 3 ein vierter Werkzeugabschnitt (9) bereitgestellt, welcher ein Halteelement (9b) aufweist, an welchem in dieser bevorzugten Ausführungsform ein Dichtelement (200) angebracht wird, insbesondere aufgesteckt. Nun wird die Stirnfläche (9a) des vierten Werkzeugabschnitts (9) mit der ersten Stirnfläche (2a) des ersten Werkzeugabschnitts (2) in Kontakt gebracht.

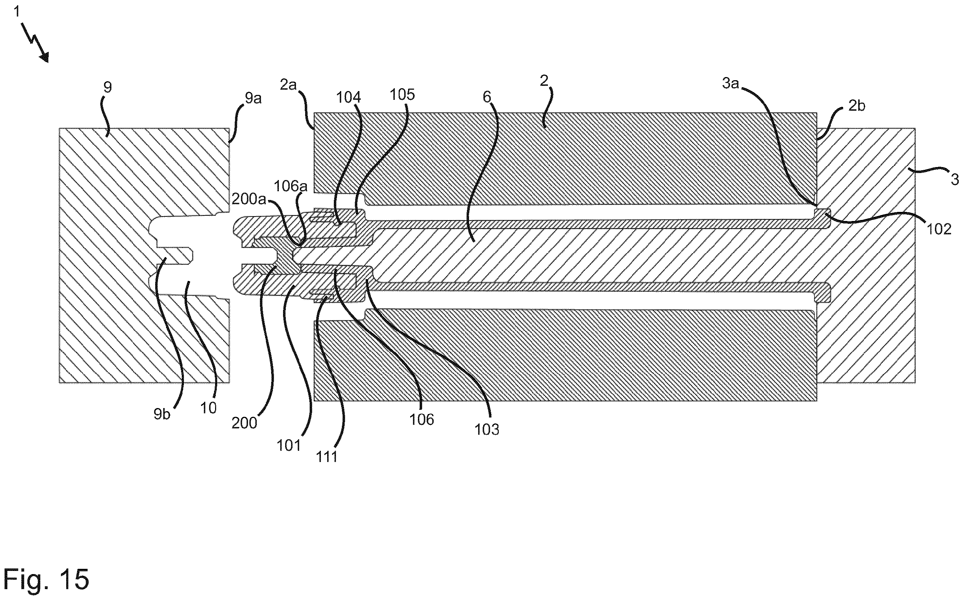

Figur 15 zeigt eine weitere Schnittdarstellung des Spritzgusswerkzeugs aus Figur 4, wobei sich in dieser Darstellung die Stirnflächen (2a, 9a) bereits kontaktieren und so mit dem Spritzgusskern (6) des zweiten Werkzeugabschnitts (3) und dem ausgebildeten Spritzenkörper (102) eine zweite Kavität (11) bilden. Beim Kontaktieren der Stirnflächen (2a, 9a) wird das Dichtelement (200) fest gegen das hohlzylindrische Endstück (106) des Spritzenkörpers (102) gedrückt. Dabei ist der Abstand (a) in der ersten axialen Richtung (X) zwischen dem Halteelement (9b) und den Ende des ersten Spritzgusskerns (6) kleiner oder gleich der Dicke (d) des Dichtelements (200). Somit kontaktieren sich die Stirnfläche (200a) des Dichtelements (200) und die Stirnfläche (106a) des hohlzylindrischen Endstücks (106). Daraufhin kann das zweite Kunststoffmaterial in die zweite Kavität (11) eingespritzt werden. Im Gegensatz zu der vorhergehenden Ausführungsform ohne Dichtelement (200) wird nun das hohlzylindrische Endstück (106) durch das Dichtelement (200) und nicht durch das zweite Kunststoffmaterial abgedichtet.

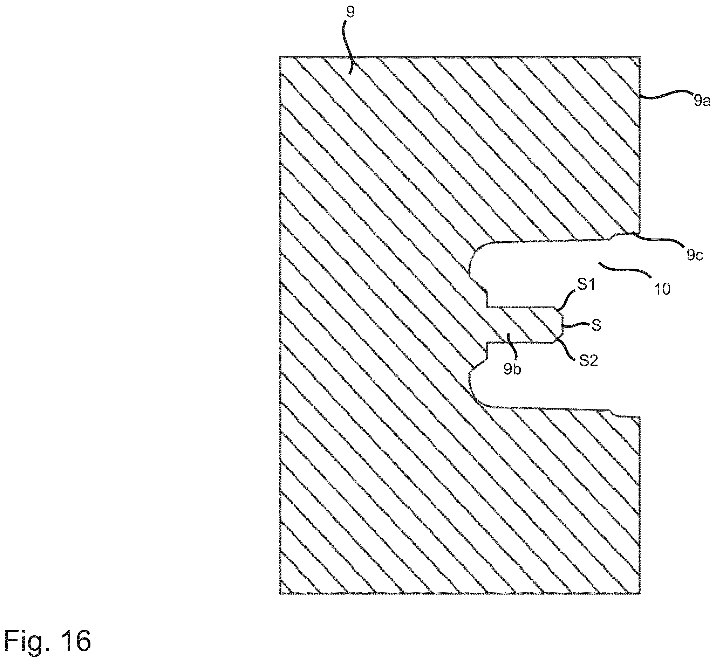

Figur 16 zeigt eine weitere Schnittdarstellung des Spritzgusswerkzeugs (1) einer bevorzugten Ausführungsform, nach Beendigung des Verfahrens. In dieser Darstellung werden der erste Werkzeugabschnitt (2) und der vierte Werkzeugabschnitt (9) von der Spritze (100) entformt, so dass die Spritze (100) entnommen werden kann, wobei die Spritze (100) bei der Entnahme von dem zweiten Werkzeugabschnitt (3) abgezogen wird.

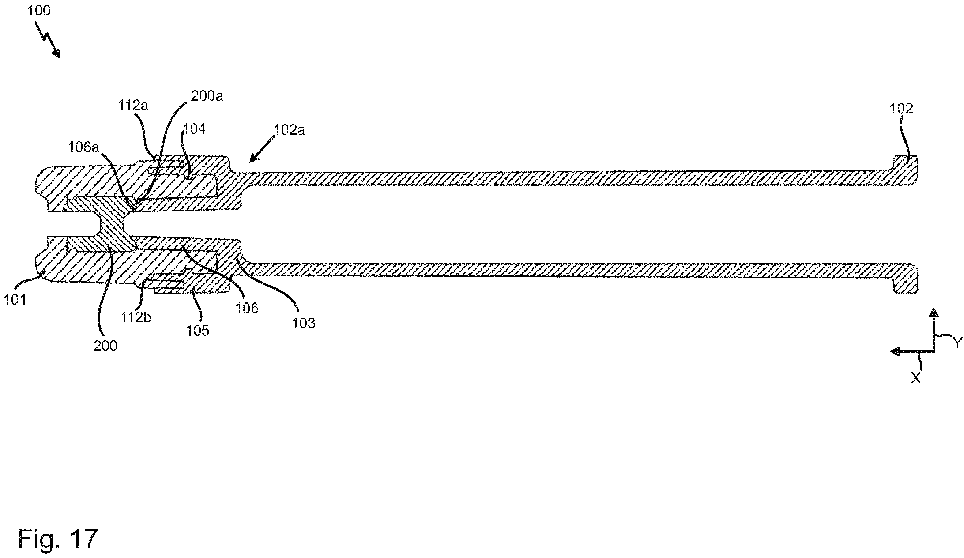

Figur 17 zeigt eine Schnittdarstellung des vierten Werkzeugabschnitts (9). Dieser Werkzeugabschnitt (9) weist eine Stirnfläche (9a) sowie ein Halteelement (9b) auf. Das Halteelement ragt dabei in den Formhohlraum (10) des vierten Werkzeugabschnitts (9) hinein. An diesem Halteelement (9b) kann in einer bevorzugten Ausführungsform ein Dichtelement (200) angebracht werden. Ebenso könnte in diesem Werkzeugabschnitt (9) auf das Halteelement (9b) verzichtet werden, wenn kein Dichtelement (200) in der Spritze (100) vorhanden sein soll. Weiterhin weist der Werkzeugabschnitt (9) Vertiefungen (9c) auf, durch welche die Formhälfte (9) an die Form des Spritzenkörpers (102) angepasst ist und so die Vorsprünge (111) des Verschlusselements (101) durch die Aussparungen (112) beim Einspritzen des zweiten Kunstsstoffmaterials gebildet werden können. Weiterhin ist das Halteelement (9b) sowie das Dichtelement (200) im Wesentlichen rotationssymmetrisch ausgestaltet. Das Halteelement (9b) weist dabei an einer Vorderseite (S) eine erste Schräge (S1) und eine zweite Schräge (S2) auf, welche das Halteelement (9b) in einem Endbereich verjüngen und durch welche das Anbringen und die Positionierung des Dichtelements (200) an dem Halteelement (9b) erleichtert wird.

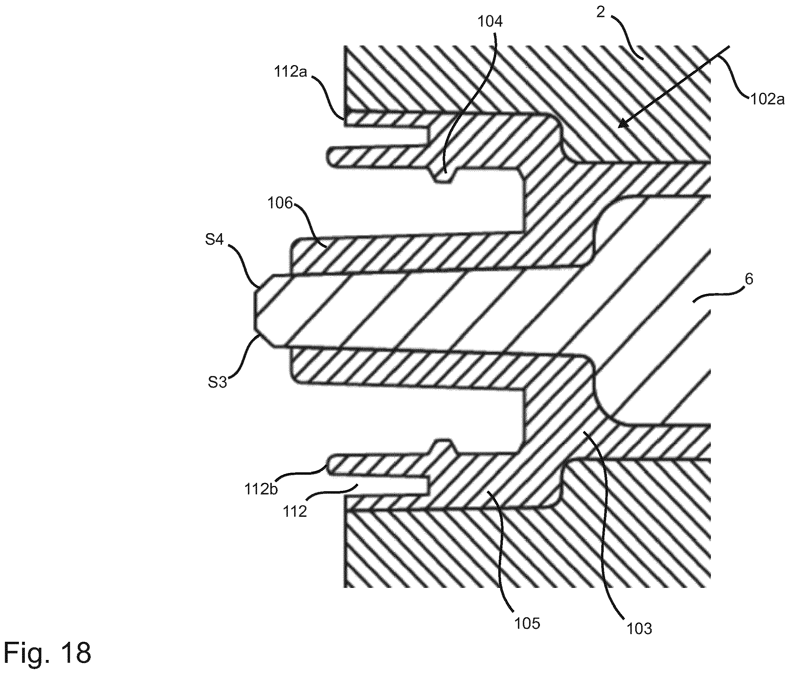

Figur 18 zeigt eine Schnittdarstellung einer Spritze (100), hergestellt nach dem zuvor genannten Verfahren. Hierbei wurde durch das Spritzgussverfahren ein hohlzylindrischer Spritzenkörper (102) geformt, welcher nun befüllt werden kann. Dieser Spritzenköper (102) weist an seinem distalen Ende (102a) einen Endbereich (103) auf, welcher ein Anschlusselement (105) umfasst. An diesem Anschlusselement (105) sind, wie bereits erwähnt, ein Innengewinde (104) sowie Aussparungen (112) ausgebildet. In diese Aussparungen (112) greifen nun Vorsprünge (111) des Verschlusselements (101) ein. Weiterhin ist ein Dichtelement (200) vorgesehen, welches derart von dem zweiten Kunststoffmaterial umschossen ist, dass weiterhin eine Abdichtung des hohlzylinderischen Endstücks (106) gegenüber der Umgebung gewährleistet ist.

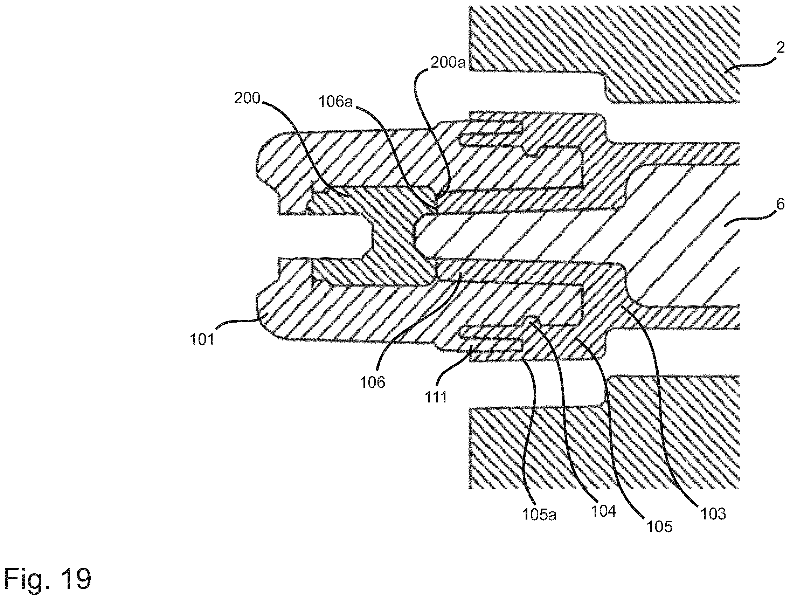

Figur 19 zeigt einen vergrößerten Ausschnitt des distalen Endes (102a) des Spritzenkörpers (102). Hier ist insbesondere zu erkennen, dass die Aussparung (112) von einem inneren (112b) und einem äußeren Wandungsabschnitt (112a) zumindest in der zweiten axialen Richtung (Y) umrandet ist. Somit ist die Aussparung (112) einseitig in einer ersten axialen Richtung (X) geöffnet und in einer zweiten axialen Richtung (Y) durch die Wandungsabschnitte (112a, 112b) begrenzt und weist damit einen im Wesentlichen U-förmigen Querschnitt auf. Der innere Wandungsabschnitt (112b) erstreckt sich dabei in der ersten axialen Richtung (X) weiter als der äußere Wandungsabschnitt (112a). Der zweite Spritzgusskern (6) ragt hierbei über das hohlzylindrische Endstück (106) hinaus und weist dabei eine erste Schräge (S3) und eine zweite Schräge (S4) auf, welche die Positionierung des Dichtelements (200) (hier nicht gezeigt) erleichtern.

Figur 20 zeigt ebenfalls einen vergrößerten Ausschnitt des distalen Endes (102a) des Spritzenkörpers (102). In dieser Darstellung wurde das Verschlusselement (101) bereits an dem Anschlusselement (105) angeformt. Das Dichtelement (200) wird durch das ausgestaltete Verschlusselement (101) weiterhin an das hohlzylindrische Endstück (106) des Spritzenkörpers (102) gedrückt. Weiterhin bildet der äußere Wandungsabschnitt (112a) einen Teil der Außenfläche (105a) des Anschlusselements (105).

### Bezugszeichenliste

- 1: Spritzgusswerkzeug
- 2: erster Werkzeugabschnitt
- 2a: Stirnfläche des ersten Werkzeugabschnitts
- 2b: Stirnfläche des ersten Werkzeugabschnitts
- 3: zweiter Werkzeugabschnitt
- 3a: Stirnfläche des zweiten Werkzeugabschnitts
- 4: dritter Werkzeugabschnitts
- 4a: Stirnfläche des dritten Werkzeugabschnitts
- 5: Formhohlraum des ersten Werkzeugabschnitts
- 5a: erste Öffnung des Formhohlraums
- 5b: zweite Öffnung des Formhohlraums
- 5c: innere Wandung des Formhohlraums
- 6: erster Spritzgusskern
- 7: zweiter Spritzgusskern
- 7a: Außenbereich des zweiten Spritzgusskerns
- 7b: Vorsprünge und Ausnehmungen am Außenbereich des zweiten Spritzgusskerns
- 8: erste Kavität
- 9: vierter Werkzeugabschnitt
- 9a: Stirnfläche des vierten Werkzeugabschnitts
- 10: Formhohlraum des vierten Werkzeugabschnitts
- 10a: innere Wandung des Formhohlraums des vierten Werkzeugabschnitts
- 10b: Ausnehmungen und Vorsprünge an innerer Wandung des Formhohlraums des vierten Werkzeugabschnitts
- 11: zweite Kavität
- 12: erster zylindrischer Abschnitt
- 12a: erster Durchmesser
- 12b: äußere Wandung des ersten zylindrischen Abschnitts
- 13: zweiter zylindrischer Abschnitt
- 13a: zweiter Durchmesser
- 13b: äußere Wandung
- 14: Formhohlraum des zweiten Spritzgusskerns
- 14a: innere Wandung des Formhohlraums des zweiten Spritzgusskerns
- 15: Ausnehmung
- 16: dritte Kavität
- 17: vierte Kavität
- 18: erste Einspritzdüse
- 19: zweite Einspritzdüse
- 20: Ausnehmung des zweiten Werkzeugabschnitts
- 21: maximale axiale Position
- 100: Spritze
- 101: Verschlusselement
- 102: hohlzylindrischer Spritzenkörper
- 102a: distales Ende des Spritzenkörpers
- 102b: proximales Ende des Spritzenkörpers
- 102c: Innendurchmesser des Spritzenkörpers
- 102d: Außendurchmesser des Spritzenkörpers
- 103: Endbereich
- 104: Innengewinde
- 105: Anschlusselement
- 106: Endstück
- 106a: äußere Wandung des Endstücks
- 106b: Innendurchmesser des Endstücks
- 106c: Außendurchmesser des Endstücks
- 107: Fingerauflage
- 108: Kanal des Endstücks
- 109: Kolben
- 109a: Gewinde-Sackbohrung des Kolbens
- 110: Dichtabschnitt
- X: axiale Richtung
- Y: zweite Richtung
- X1: Dichtabschnitt
- 100: Spritze
- 101: Verschlusselement
- 102: Spritzenkörper
- 102a: distales Ende des Spritzenkörpers
- 103: Endbereich
- 104: Innengewinde
- 105: Anschlusselement
- 105a: Außenfläche des Anschlusselements
- 106: Endstück
- 106a: Stirnfläche des Endstücks
- 111: Vorsprung
- 112: Aussparung an dem Anschlusselement
- 112a: äußerer Wandungsabschnitt der Aussparung
- 112b: innerer Wandungsabschnitt der Aussparung
- 200: Dichtelement
- 200a: Stirnfläche des Dichtelements
- a: Abstand zwischen Halteelement und dem ersten Spritzgusskern
- d: Dicke des Dichtelements
- S1: erste Schräge der Vorderseite des Halteelements
- S2: zweite Schräge der Vorderseite des Halteelements
- S3: erste Schräge des ersten Spritzgusskerns
- S4: zweite Schräge des zweiten Spritzgusskerns
- X: erste achsiale Richtung
- Y: zweite achsiale Richtung

## Patentansprüche

1. Spritzgießverfahren zum Herstellen einer Spritze (100) mit einem integrierten Verschlusselement (101) umfassend folgende Verfahrensschritte:
a) Bereitstellen eines Spritzgusswerkzeugs (1), welches einen ersten (2), einen zweiten (3) und einen dritten Werkzeugabschnitt (4) umfasst, wobei der erste Werkzeugabschnitt (2) einen sich entlang einer axialen Richtung (X) erstreckenden beidseitig offenen Formhohlraum (5) aufweist und wobei der zweite Werkzeugabschnitt (3) einen ersten Spritzgusskern (6) und der dritte Werkzeugabschnitt (4) einen zweiten Spritzgusskern (7) aufweist;
b) Schließen des Spritzgusswerkzeugs (1), so dass der erste Werkzeugabschnitt (2) den zweiten (3) und dritten Werkzeugabschnitt (4) kontaktiert und der erste (6) und zweite Spritzgusskern (7) durch jeweils eine Öffnung (5a, 5b) in den Formhohlraum (5) des ersten Werkzeugabschnitts (2) in diesen eintreten und sich schließlich kontaktieren, wodurch diese Werkzeugabschnitte (2, 3, 4) eine erste Kavität (8) bilden;
c) Einspritzen eines ersten Kunststoffmaterials in die erste Kavität (8), wodurch ein hohlzylindrischer Spritzenkörper (102), an dessen distalen Ende (102a) ein Endbereich (103) angeordnet ist, ausgebildet wird, wobei der Endbereich (103) ein mit einem Innengewinde (104) versehenes Anschlusselement (105) und ein hohlzylindrisches Endstück (106), welches von dem Anschlusselement (105) zumindest abschnittsweise umrandet ist, aufweist;
d) Abkühlen der Werkzeugabschnitte (2, 3, 4), wodurch der Spritzenkörper (102) abkühlt und aushärtet;
e) Kontaktieren des ersten Werkzeugabschnitts (2) mit einen einseitig geschlossenen Formhohlraum (9) versehenen vierten Werkzeugabschnitt, wodurch eine zweite Kavität (11) an dem distalen Ende (102a) des Spritzenkörpers (102) gebildet wird;
f) Einspritzen eines zweiten Kunststoffmaterials in die zweite Kavität (11), wodurch das Verschlusselement (101) an das Anschlusselement (105) angeformt wird, wobei das erste und das zweite Kunststoffmaterial keine stoffschlüssige Verbindung eingehen.

2. Verfahren nach Anspruch 1,
**dadurch gekennzeichnet, dass**
der Endbereich (103) ein dem einem Innengewinde (104) und wenigstens einer Aussparung (112) versehenes Anschlusselement (105) aufweist, wobei durch das Einspritzen des zweiten Kunststoffmaterials in die zweite Kavität (11), das Verschlusselement (101) an das Anschlusselement (105) angeformt wird, wodurch in der wenigstens einen Aussparung (112) ein Vorsprung (111) ausgebildet wird.

3. Verfahren nach zumindest einem der vorhergehenden Ansprüche,
**dadurch gekennzeichnet, dass**
der erste (6) und zweite Spritzgusskern (7) sich entlang der axialen Richtung (X) er-strecken, wobei der erste Spritzgusskern (6) einen ersten zylindrischen Abschnitt (12) mit einem ersten Durchmesser (12a) und einen zweiten zylindrischen Abschnitt (13) mit einem zweiten kleineren Durchmesser (13a) aufweist, wobei der erste (12) und der zweite zylindrische Abschnitt (13) in axialen Richtung (X) angrenzend sind, und wobei der zweite Spritzgusskern (7) einen in axialer Richtung (X) einseitig offenen Formholraum (14) und eine Ausnehmung (15) an einer den Formholraum (14) in axialer Richtung (X) begrenzenden Innenwand (14a) aufweist.

4. Verfahren nach Anspruch 3,
**dadurch gekennzeichnet, dass**
in Verfahrensschritt b) eine innere Wandung (5c) des Formhohlraums (5) des ersten Werkzeugabschnitts (2) und eine äußere Wandung (12b) des ersten zylindrischen Abschnitts (12) des ersten Spritzgusskerns (6) einen ersten Abschnitt (8a) der ersten Kavität (8) bilden, mittels welchem der hohlzylindrischen Spritzenkörper (102) gebildet wird, wobei bei dem Kontaktieren der beiden Spritzgusskerne (6, 7) in Verfahrensschritt b) der zweite zylindrische Abschnitt (13) des ersten Spritzgusskerns (6) in der Ausnehmung (15) des zweiten Spritzgusskerns (7) teilweise aufgenommen wird, wobei durch den Formholraum (14) des zweiten Spritzgusskerns (7) und den zweiten zylindrischen Abschnitt (13) des ersten Spritzgusskerns (6) eine dritte Kavität (16) gebildet wird, mittels welcher das Endstück (106) ausgebildet wird ,wobei das mit dem Innen-gewinde (104) versehene Anschlusselement (105) mittels einer vierten Kavität (17) gebildet wird, welche durch einen Außenbereich (7a) des zweiten Spritzgusskerns (7) und der inneren Wandung (5c) des Formhohlraums (5) des ersten Werkzeugabschnitts (2) gebildet wird.

5. Verfahren nach einem der vorhergehenden Ansprüche,
**dadurch gekennzeichnet, dass**
zwischen den Verfahrensschritten d) und e) der zweite Spritzgusskern (7) von dem Anschlusselement (105) des Spritzenkörpers (102) entformt wird und der dritte Werkzeugabschnitt (4) von dem ersten Werkzeugabschnitt (2) entfernt wird, wobei der zweite Spritzgusskern (7) durch eine Drehbewegung von dem Innengewinde (104) des Anschlusselements (105) entformt wird.

6. Verfahren nach einem der vorhergehenden Ansprüche,
**dadurch gekennzeichnet, dass**
das erste Kunststoffmaterial durch eine erste Einspritzdüse (18), welche in dem zweiten Werkzeugabschnitt (3) angeordnet ist, eingespritzt wird und das zweite Kunst-stoffmaterial durch eine zweite Einspritzdüse (19), welche in dem vierten Werkzeug-abschnitt (9) angeordnet ist eingespritzt wird, wobei der Formhohlraum (5) des ersten Werkzeugabschnitts (2) eine erste Öffnung (5a) aufweist, in welche der erste Spritzgusskern (6) in Verfahrensschritt b) eintritt, wobei der zweite Werkzeugabschnitt (3) eine Ausnehmung (20) aufweist, welche zur ersten Kavität (8) hin offen ist und mittels welcher eine Fingerauflage (107) am proximalen Ende (102b) des Spritzenkörpers (102) gebildet wird, wobei die erste Einspritzdüse (18) mit dieser Ausnehmung (20) verbunden ist.

7. Verfahren nach einem der vorhergehenden Ansprüche,
**dadurch gekennzeichnet, dass**
der erste (2), zweite (3), dritte (4) und vierte Werkzeugabschnitt (9) individuell erwärmt und gekühlt werden, wobei die Erwärmung mittels eines Lasers erfolgt und die Kühlung mittels eines Kühlmittels, wobei das Kühlmittel Wasser und/oder Stickstoff und/oder CO2 enthält.

8. Verfahren nach einem der vorhergehenden Ansprüche,
**dadurch gekennzeichnet, dass**
der erste Spritzgusskern (6) teleskopartig ausgestaltet ist, wobei der zweite zylindrische Abschnitt (13) relativ zu dem ersten Abschnitt (12) verlagerbar ist, wobei der zweite zylindrische Abschnitt (13) in den Verfahrensschritten b) bis d) sich in einer maximalen axialen Position (21) befindet, so dass der zweite zylindrische Abschnitt (13) den zweiten Spritzgusskern (7) kontaktiert, wobei zwischen Verfahrensschritt d) und e) der zweite Abschnitt (13) in axialer Richtung (X) von dem dritten Werkzeugabschnitt (4) weg verlagert wird, wobei in dem nachfolgenden Verfahrensschritt f) der zweite Kunststoff in den Kanal (108) des Endstücks (106) eintritt, wodurch ein Ab-dichtung des Kanals (108) erzeugt wird.

9. Verfahren nach einem der vorhergehenden Ansprüche,
**dadurch gekennzeichnet, dass**
das erste und das zweite Kunststoffmaterial unterschiedliche Materialien sind oder das erste und das zweite Kunststoffmaterial gleiche Materialien sind, wobei das erste Kunststoffmaterial ein Polymer-Kunststoff, bevorzugt ein Polyolefin, beispielsweise Polypropylen oder Polyethylen, besonders bevorzugt ein Cyclo-Olefines Polymer (COP) beziehungsweise ein Cyclo-Olefines Co-Polymer (COC) ist und das zweite Kunststoffmaterial ein thermoplastisches Elastomer oder ein Polymer-Kunststoff, bevorzugt ein Polyolefin ist.

10. Verfahren nach einem der Ansprüche 2 bis 9,
**dadurch gekennzeichnet, dass**
die zumindest eine Aussparung (112) von einem äußeren (112a) und einem inneren (112b) Wandungsabschnitt umrandet ist, wobei der äußere Wandungsabschnitt (112a) einen Teil der Außenfläche (105a) des Anschlusselements (105) bildet.

11. Verfahren nach einem der vorangegangenen Ansprüche,
**dadurch gekennzeichnet, dass**
die Steigung des Innengewindes (104) des Anschlusselements (105) der Neigung des wenigstens einen Vorsprungs (111) entspricht.

12. Verfahren nach einem der vorangegangenen Ansprüche 2 bis 11,
**dadurch gekennzeichnet, dass**
zwei, bevorzugter drei und besonders bevorzugt mehr als drei Aussparungen (112) an dem Anschlusselement (105) und dazu komplementäre Vorsprünge (111) an dem Verschlusselement (101) ausgebildet werden.

13. Verfahren nach einem der vorangegangenen Ansprüche,
**dadurch gekennzeichnet, dass**
nach dem Verfahrensschritt d) in einem weiteren Verfahrensschritt d2) ein Dichtelement (200) an dem vierten Werkzeugabschnitt (9) angebracht wird, wobei der vierte Werkzeugabschnitt (9) ein in den Formhohlraum (10) des vierten Werkzeugabschnitts (9) hineinragendes Halteelement (9b) aufweist, welches zum Halten des Dichtelements (200) ausgestaltet ist, wobei das Dichtelement (200) in dem Verfahrensschritt e) an das hohlzylindrische Endstück (106) des Spritzenkörpers (102) gedrückt wird, wobei das Dichtelement (200) nach dem Verfahrensschritt f) von dem zweiten Kunststoffmaterial umschlossen wird, während das Dichtelement (200) nach dem Entformen des vierten Werkzeugabschnitts (9) weiterhin an das hohlzylindrische Endstück (106) des Spritzenkörpers (102) gedrückt wird.

14. Spritze mit einem integrierten Verschlusselement (101) mit wenigstens einem an dem Verschlusselement (101) angeordneten Vorsprung (111) hergestellt nach einem Verfahren gemäß einem der vorhergehenden Ansprüche, umfassend
einen hohlzylindrischen Spritzenkörper (102), an dessen distalen Ende (102a) ein Endbereich (103) angeordnet ist, wobei der Endbereich (103) ein mit einem Innengewinde (104) versehenes Anschlusselement (105) und ein hohlzylindrisches Endstück (106), welches von dem Anschlusselement (105) zumindest abschnittsweise umrandet ist, aufweist, wobei an dem Anschlusselement (105) wenigstens eine Aussparung (112) ausgebildet ist, wobei das Verschlusselement (101) an das Anschlusselement (105) angeformt ist, wodurch in der wenigstens einen Aussparung (112) der Vorsprung (111) ausgebildet ist, wobei sich der Vorsprung (111) entlang einer ersten axialen Richtung (X) erstreckt.

15. Spritze nach Anspruch 14,
**dadurch gekennzeichnet, dass**
an dem Verschlusselement (101) ein Dichtelement (200) angeordnet ist.

## Claims

1. Injection moulding process for producing a syringe (100) with an integrated closure element (101) comprising the following process steps:
a) providing an injection moulding tool (1) which comprises a first (2), a second (3) and a third tool portion (4), wherein the first tool portion (2) has a mould cavity (5) open at both sides and extending along an axial direction (X), and wherein the second tool portion (3) has a first injection moulding core (6) and the third tool portion (4) has a second injection moulding core (7);
b) closing the injection moulding tool (1) such that the first tool portion (2) contacts the second (3) and third tool portion (4), and the first (6) and second injection moulding core (7) each enter the mould cavity (5) of the first tool portion (2) through an opening (5a, 5b) and finally contact each other, as a result of which these tool portions (2, 3, 4) form a first cavity (8);
c) injecting a first plastic material into the first cavity (8), as a result of which a hollow cylindrical syringe body (102) is formed with an end region (103) at its distal end (102a), wherein the end region (103) has an attachment element (105), provided with an inner thread (104), and a hollow cylindrical end piece (106) which is at least partially bordered by the attachment element (105);
d) cooling the tool portions (2, 3, 4), as a result of which the syringe body (102) cools and hardens;
e) bringing the first tool portion (2) into contact with a fourth tool portion provided with a mould cavity (9) closed at one end, as a result of which a second cavity (11) is formed at the distal end (102a) of the syringe body (102);
f) injecting a second plastic material into the second cavity (11), as a result of which the closure element (101) is integrally formed on the attachment element (105), wherein the first and the second plastic material do not enter into a cohesive connection.

2. Method according to claim 1,
**characterised in that**
the end region (103) has an attachment element (105) provided with an inner thread (104) and at least one recess (112), wherein by injecting the second plastic material into the second cavity (11), the closure element (101) is integrally formed on the attachment element (105), as a result of which a protrusion (111) is formed in the at least one recess (112).

3. Method according to at least one of the preceding claims,
**characterised in that**
the first (6) and the second injection moulding core (7) extend along the axial direction (X), wherein the first injection moulding core (6) comprises a first cylindrical portion (12) with a first diameter (12a) and a second cylindrical portion (13) with a second, smaller diameter (13a), said first (12) and said second cylindrical portion (13) being adjacent in the axial direction (X), and wherein the second injection moulding core (7) has a mould cavity (14) open on one side in the axial direction (X) and a recess (15) on an inner wall (14a) bordering the mould cavity (14) in the axial direction (X).

4. Method according to claim 3,
**characterised in that**
in process step b), an inner wall (5c) of the mould cavity (5) of the first tool portion (2) and an outer wall (12b) of the first cylindrical portion (12) of the first injection moulding core (6) form a first portion (8a) of the first cavity (8), by means of which the hollow cylindrical syringe body (102) is formed, wherein, with the contacting of the two injection moulding cores (6, 7) in process step b), the second cylindrical portion (13) of the first injection moulding core (6) is received in part in the recess (15) of the second injection moulding core (7), wherein a third cavity (16) is formed by the mould cavity (14) of the second injection moulding core of (7) and the second cylindrical portion (13) of the first injection moulding core (6), by means of which the end piece (106) is formed, wherein the attachment element (105) provided with the inner thread (104) is formed by means of a fourth cavity (17), which is formed by an outer region (7a) of the second injection moulding core (7) and the inner wall (5c) of the mould cavity (5) of the first tool portion (2).

5. Method according to any of the preceding claims,
**characterised in that**
between process steps d) and e), the second injection moulding core (7) is demoulded from the attachment element (105) of the syringe body (102) and the third tool portion (4) is removed from the first tool portion (2), wherein the second injection moulding core (7) is demoulded by a rotational movement of the inner thread (104) of the attachment element (105).

6. Method according to any of the preceding claims,
**characterised in that**
the first plastic material is injected through a first injection nozzle (18) arranged in the second tool portion (3), and the second plastic material is injected through a second injection nozzle (19) arranged in the fourth tool portion (9), wherein the mould cavity (5) of the first tool portion (2) has a first opening (5a) into which the first injection mould core (6) enters in process step b), wherein the second tool portion (3) has a recess (20) that is open to the first cavity (8) and by means of which a finger rest (107) is formed at the proximal end (102b) of the syringe body (102), wherein the first injection nozzle (18) is connected to this recess (20).

7. Method according to any of the preceding claims,
**characterised in that**
the first (2), second (3), third (4) and fourth tool portions (9) are individually heated and cooled, wherein the heating takes place by means of a laser and the cooling by means of a coolant, wherein the coolant contains water and/or nitrogen and/or CO₂.

8. Method according to any of the preceding claims,
**characterised in that**
the first injection moulding core (6) is telescopically designed, wherein the second cylindrical portion (13) is displaceable relative to the first portion (12), wherein the second cylindrical portion (13) in process steps b) to d) is in a maximum axial position (21) such that the second cylindrical portion (13) contacts the second injection moulding core (7), wherein, between process steps d) and e), the second portion (13) is displaced in the axial direction (X) away from the third tool portion (4), wherein, in the following process step f), the second plastic material enters the channel (108) of the end piece (106), as a result of which a sealing of the channel (108) is created.

9. Method according to any of the preceding claims,
**characterised in that**
the first and the second plastic material are different materials or the first and the second plastic material are similar materials, wherein the first plastic material is a polymer plastic material, preferably a polyolefin, for example polypropylene or polyethylene, particularly preferably a cyclic olefin polymer (COP) or a cyclic olefin copolymer (COC), and the second plastic material is a thermoplastic elastomer or a polymer plastic material, preferably a polyolefin.

10. Method according to any of claims 2 to 9,
**characterised in that**
the at least one recess (112) is surrounded by an outer (112a) and an inner (112b) wall portion, wherein the outer wall portion (112a) forms a part of the outer surface (105a) of the attachment element (105).

11. Method according to any of the preceding claims,
**characterised in that**
the pitch of the inner thread (104) of the attachment element (105) corresponds to the inclination of the at least one protrusion (111).

12. Method according to any of the preceding claims 2 to 11,
**characterised in that**
two, preferably three, and particularly preferably more than three recesses (112) are formed on the attachment element (105), and protrusions (111) complementary thereto are formed on the closure element (101).

13. Method according to any of the preceding claims,
**characterised in that**
after step d), in a further process step d2), a sealing element (200) is mounted on the fourth tool portion (9), said fourth tool portion (9) having a holding element (9b) projecting into the mould cavity (10) of the fourth tool portion (9) that is designed to hold the sealing element (200), wherein the sealing element (200) in process step e) is pressed against the hollow cylindrical end piece (106) of the syringe body (102), wherein the sealing element (200) after process step f) is surrounded by the second plastic material, while the sealing element (200) after the demoulding of the fourth tool portion (9) is still pressed against the hollow cylindrical end piece (106) of the syringe body (102).

14. Syringe having an integrated closure element (101) with at least one protrusion (111) arranged on the closure element (101) produced in accordance with a method according to any one of the preceding claims, comprising
a hollow cylindrical syringe body (102) having at its distal end (102a) an end region (103), wherein the end region (103) comprises an attachment element (105) with an inner thread (104) and a hollow cylindrical end piece (106), which is at least partially bordered by the attachment element (105), wherein at least one recess (112) is formed on the attachment element (105), wherein the closure element (101) is integrally formed with the attachment element (105), whereby the protrusion (111) is formed in the at least one recess (112), the projection (111) extending along a first axial direction (X).

15. Syringe according to claim 14,
**characterised in that**
a sealing element (200) is arranged on the closure element (101).

## Revendications

1. Procédé de moulage par injection pour la fabrication d'une seringue (100) ayant un élément de fermeture intégré (101), comportant les étapes de procédé suivantes :
a) mettre à disposition un outil de moulage par injection (1), lequel comporte une première (2), une deuxième (3) et une troisième (4) section d'outil, la première section d'outil (2) présentant une cavité de moule (5) ouverte sur deux côtés, s'étendant le long d'une direction axiale (X), et la deuxième section d'outil (3) présentant un premier noyau de moulage par injection (6) et la troisième section d'outil (4), un second noyau de moulage par injection (7) ;
b) fermer l'outil de moulage par injection (1) de telle sorte que la première section d'outil (2) vient en contact avec la deuxième (3) et la troisième (4) section d'outil et le premier (6) et le second (7) noyau de moulage par injection pénètrent, par une ouverture respective (5a, 5b) dans la cavité de moule (5) de la première section d'outil (2), dans ladite cavité, et finalement viennent en contact l'un avec l'autre, ce par quoi ces sections d'outil (2, 3, 4) forment une première cavité (8) ;
c) injecter une première matière plastique dans la première cavité (8), ce par quoi un corps de seringue cylindrique creux (102) est formé, à l'extrémité distale (102a) duquel une région d'extrémité (103) est disposée, la région d'extrémité (103) présentant un élément de raccordement (105) muni d'un filetage interne (104), et une pièce d'extrémité cylindrique creuse (106), laquelle est au moins par sections entourée par l'élément de raccordement (105) ;
d) refroidir les sections d'outil (2, 3, 4), ce par quoi le corps de seringue (102) refroidit et durcit ;
e) mettre en contact la première section d'outil (2) avec une quatrième section d'outil munie d'une cavité de moule (9) fermée sur un côté, ce par quoi une seconde cavité (11) est formée à l'extrémité distale (102a) du corps de seringue (102) ;
f) injecter une seconde matière plastique dans la seconde cavité (11), ce par quoi l'élément de fermeture (101) est moulé sur l'élément de raccordement (105), la première et la seconde matière plastique ne parvenant pas à un assemblage par liaison de matière.

2. Procédé selon la revendication 1,
**caractérisé par le fait que**
la région d'extrémité (103) présente un élément de raccordement (105) pourvu d'un filetage interne (104) et d'au moins un évidement (112), où, par l'injection de la seconde matière plastique dans la seconde cavité (11), l'élément de fermeture (101) est moulé sur l'élément de raccordement (105), ce par quoi une saillie (111) est formée dans ledit au moins un évidement (112).

3. Procédé selon au moins l'une des revendications précédentes,
**caractérisé par le fait que**
le premier (6) et le second (7) noyau de moulage par injection s'étendent le long de la direction axiale (X), le premier noyau de moulage par injection (6) présentant une première section cylindrique (12) avec un premier diamètre (12a) et une seconde section cylindrique (13) avec un second diamètre plus petit (13a), la première (12) et la seconde (13) section cylindrique étant adjacentes dans la direction axiale (X), et le second noyau de moulage par injection (7) présentant une cavité de moule (14) ouverte sur un côté dans la direction axiale (X) et un évidement (15) sur une paroi interne (14a) délimitant la cavité de moule (14) dans la direction axiale (X).

4. Procédé selon la revendication 3,
**caractérisé par le fait qu'**
à l'étape de procédé b), une paroi interne (5c) de la cavité de moule (5) de la première section d'outil (2) et une paroi externe (12b) de la première section cylindrique (12) du premier noyau de moulage par injection (6) forment une première section (8a) de la première cavité (8), au moyen de laquelle le corps de seringue cylindrique creux (102) est formé, où, lors du contact des deux noyaux de moulage par injection (6, 7) à l'étape de procédé b), la seconde section cylindrique (13) du premier noyau de moulage par injection (6) est partiellement reçue dans l'évidement (15) du second noyau de moulage par injection (7), où, par la cavité de moule (14) du second noyau de moulage par injection (7) et la seconde section cylindrique (13) du premier noyau de moulage par injection (6) est formée une troisième cavité (16), au moyen de laquelle la pièce d'extrémité (106) est formée, l'élément de raccordement (105) muni du filetage interne (104) étant formé au moyen d'une quatrième cavité (17), laquelle est formée par une région externe (7a) du second noyau de moulage par injection (7) et la paroi interne (5c) de la cavité de moule (5) de la première section d'outil (2).

5. Procédé selon l'une des revendications précédentes,
**caractérisé par le fait qu'**
entre les étapes de procédé d) et e), le second noyau de moulage par injection (7) est démoulé de l'élément de raccordement (105) du corps de seringue (102) et la troisième section d'outil (4) est enlevée de la première section d'outil (2), le second noyau de moulage par injection (7) étant démoulé par un mouvement de rotation du filetage interne (104) de l'élément de raccordement (105).

6. Procédé selon l'une des revendications précédentes,
**caractérisé par le fait que**
la première matière plastique est injectée par une première buse d'injection (18), laquelle est disposée dans la deuxième section d'outil (3), et la seconde matière plastique est injectée par une seconde buse d'injection (19), laquelle est disposée dans la quatrième section d'outil (9), la cavité de moule (5) de la première section d'outil (2) présentant une première ouverture (5a), dans laquelle le premier noyau de moulage par injection (6) pénètre à l'étape de procédé b), la deuxième section d'outil (3) présentant un évidement (20), lequel est ouvert en direction de la première cavité (8) et au moyen duquel un support de doigt (107) est formé à l'extrémité proximale (102b) du corps de seringue (102), la première buse d'injection (18) étant reliée à cet évidement (20).

7. Procédé selon l'une des revendications précédentes,
**caractérisé par le fait que**
les première (2), deuxième (3), troisième (4) et quatrième (9) sections d'outil sont chauffées et refroidies individuellement, le chauffage ayant lieu au moyen d'un laser, et le refroidissement, au moyen d'un réfrigérant, le réfrigérant contenant de l'eau et/ou de l'azote et/ou du CO₂.

8. Procédé selon l'une des revendications précédentes,
**caractérisé par le fait que**
le premier noyau de moulage par injection (6) est conçu de manière télescopique, la seconde section cylindrique (13) étant déplaçable par rapport à la première section (12), la seconde section cylindrique (13) se trouvant dans une position axiale maximale (21) dans les étapes de procédé b) à d), de telle sorte que la seconde section cylindrique (13) vient en contact avec le second noyau de moulage par injection (7), où, entre les étapes de procédé d) et e), la seconde section (13) est déplacée en s'éloignant de la troisième section d'outil (4) dans la direction axiale (X), où, à l'étape de procédé suivante f), la seconde matière plastique pénètre dans le canal (108) de la pièce d'extrémité (106), ce par quoi une étanchéité du canal (108) est réalisée.

9. Procédé selon l'une quelconque des revendications précédentes,
**caractérisé par le fait que**
la première et la seconde matière plastique sont des matières différentes ou la première et la seconde matière plastique sont des matières analogues, la première matière plastique étant une matière plastique polymère, de préférence une polyoléfine, par exemple du polypropylène ou du polyéthylène, de manière particulièrement préférée un polymère d'oléfine cyclique (COP) ou un copolymère d'oléfine cyclique (COC), et la seconde matière plastique est un élastomère thermoplastique ou est une matière plastique polymère, de préférence une polyoléfine.

10. Procédé selon l'une des revendications 2 à 9,
**caractérisé par le fait que**
ledit au moins un évidement (112) est entouré par une section de paroi externe (112a) et une section de paroi interne (112b), la section de paroi externe (112a) formant une partie de la surface externe (105a) de l'élément de raccordement (105).

11. Procédé selon l'une des revendications précédentes,
**caractérisé par le fait que**
le pas du filetage interne (104) de l'élément de raccordement (105) correspond à l'inclinaison de ladite au moins une saillie (111).

12. Procédé selon l'une des revendications précédentes 2 à 11,
**caractérisé par le fait que**
deux, plus préférentiellement trois et de manière particulièrement préférée plus de trois, évidements (112) sont disposés sur l'élément de raccordement (105) et des saillies (111) complémentaires à ceux-ci sont formées sur l'élément de fermeture (101).

13. Procédé selon l'une des revendications précédentes,
**caractérisé par le fait qu'**
après l'étape de procédé d), dans une autre étape de procédé d2), un élément d'étanchéité (200) est appliqué sur la quatrième section d'outil (9), la quatrième section d'outil (9) présentant un élément de maintien (9b) faisant saillie dans la cavité de moule (10) de la quatrième section d'outil (9), lequel est conçu pour le maintien de l'élément d'étanchéité (200), où, dans l'étape de procédé e), l'élément d'étanchéité (200) est pressé contre la pièce d'extrémité cylindrique creuse (106) du corps de seringue (102), où, après l'étape de procédé f), l'élément d'étanchéité (200) est entouré par la seconde matière plastique, tandis que l'élément d'étanchéité (200) après le démoulage de la quatrième section d'outil (9) est encore pressé contre la pièce d'extrémité cylindrique creuse (106) du corps de seringue (102).

14. Seringue comportant un élément de fermeture intégré (101) avec au moins une saillie (111) disposée sur l'élément de fermeture (101), obtenue selon un procédé selon l'une des revendications précédentes, comportant
un corps de seringue cylindrique creux (102), à l'extrémité distale (102a) duquel une région d'extrémité (103) est disposée, la région d'extrémité (103) présentant un élément de raccordement (105) muni d'un filetage interne (104) et une pièce d'extrémité cylindrique creuse (106), laquelle est au moins par sections entourée par l'élément de raccordement (105), au moins un évidement (112) étant formé sur l'élément de raccordement (105), l'élément de fermeture (101) étant moulé sur l'élément de raccordement (105), ce par quoi la saillie (111) est formée dans ledit au moins un évidement (112), la saillie (111) s'étendant le long d'une première direction axiale (X).

15. Seringue selon la revendication 14,
**caractérisée par le fait qu'**
un élément d'étanchéité (101) est disposé sur l'élément de fermeture (101).
